# EUROPEAN PATENT APPLICATION

(11) **EP 4 617 256 A1**
(43) Date of publication of application: **17.09.2025**
(21) Application number: 22964927.2
(22) Date of filing: 21.11.2022
(51) Int. Cl.: C07C 211/55, C07C 209/36, C08K 5/18, C08L 21/00

(54) **RUBBER ANTI-AGING AGENT AND PREPARATION METHOD THEREFOR**

(30) Priority: 10.11.2022 CN 202211408401
(71) Applicant: Sennics Co., Ltd., China (Shanghai) Pilot Free Trade Zone 200120 (CN)
(72) Inventor: ZHOU, Xiaoyin, Shanghai 200120 (CN); GUO, Xiangyun, Shanghai 200120 (CN); XING, Jinguo, Shanghai 200120 (CN); ZHANG, Jiaqiang, Shanghai 200120 (CN)
(74) Representative: V.O.
(86) International application number: PCT/CN2022/133176
(87) International publication number: WO 2024/098460

(57) **Abstract**

A rubber anti-aging agent having a structure represented by formula A and a preparation method therefor. In formula A, R, Rₐ and R_{b} are as defined in the description. The rubber anti-aging agent can endow rubber with good thermo-oxidative aging resistance, ozone aging resistance, discoloration resistance and durability.

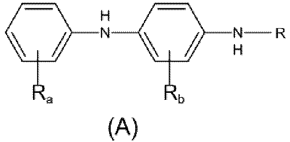

## Description

### TECHNICAL FIELD

The present invention belongs to the field of antidegradants, specifically relates to rubber antidegradants and preparation method therefor.

### BACKGROUND OF THE INVENTION

At present, p-phenylenediamine compounds are commonly used in rubber products, especially tires, as antidegradants. P-phenylenediamine compounds used as the antidegradants include dialkyl-p-phenylenediamine, alkylaryl-p-phenylenediamine, and diaryl-p-phenylenediamine, of which the most widely used antidegradant is 6PPD (N-(1,3-dimethylbutyl)-N'-phenyl-p-phenylenediamine). Other antidegradants include IPPD (N-isopropyl-N'-phenyl-p-phenylenediamine), 77PD (N,N'-bis(1,4-dimethylpentyl)-p-phenylenediamine), DTPD (a mixture of diphenyl-p-phenylenediamine, bis(tolyl)-p-phenylenediamine and phenyltolyl p-phenylenediamine), etc.

In recent years, users have paid more attention to the anti-aging durability and surface discoloration of rubber products or tires. Existing antidegradants quickly migrate to the surface during the use of rubber products or tires, which leads to surface discoloration pollution and damage to the surface of rubber products or tires. The antidegradants are also quickly consumed due to the fast migration and provide relatively poor long-lasting protection. Although non-polluting antidegradants such as CMA (N-cyclohexyl-p-methoxyaniline) have relatively good ozone aging resistance and radiation aging resistance, they have poor migration resistance and therefore provide poor long-lasting protection.

### SUMMARY OF THE INVENTION

To solve the above problems, the present invention provides a rubber antidegradant with novel structure and green synthesis process thereof. The rubber antidegradant of the present invention provides good thermal oxidative aging resistance, ozone aging resistance, discoloration resistance, and durable performance, and have little effect on the processing/vulcanization performance and pre-aging physical properties of the rubber. The present invention also provides a green method for the synthesis of the rubber antidegradant.

Specifically, the present invention provides a compound as a rubber antidegradant having a structure of Formula A: 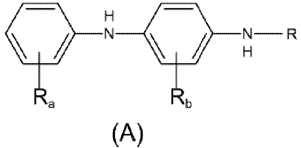
wherein R is selected from a C1-C20 chain hydrocarbon group, a C3-C20 alicyclic hydrocarbon group, a C6-C20 aryl group, and a C1-C20 alkoxy group;
Rₐ is selected from hydrogen (H), a C1-C20 alkyl, a C3-C20 cycloalkyl, phenyl, a C7-C20 alkyl phenyl, a C1-C20 alkyloxy, a C3-C20 cycloalkyloxy, and a C7-C20 alkylphenyloxy;
R_{b} is selected from hydrogen, a C1-C20 alkyl, a C3-C20 cycloalkyl, phenyl, and a C7-C20 alkyl phenyl;
and the compounds of Formula A exclude those where R_{b} is hydrogen and Rₐ is selected from hydrogen, a C1-C20 alkyl, a C3-C20 cycloalkyl, phenyl, and a C7-C20 alkyl phenyl.

In one or more embodiments, the compound of Formula A of the present invention has the following structure as shown in Formula B: 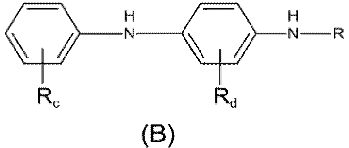
wherein R is selected from a C1-C20 chain hydrocarbon group, a C3-C20 alicyclic hydrocarbon group, and a C6-C20 aryl group;
R_{c} and R_{d} are independently selected from a C1-C10 alkyl, a C3-C10 cycloalkyl, phenyl, and a C7-C10 alkyl phenyl.

In one or more embodiments, in Formula B, R is selected from a C3-C10 branched-chain hydrocarbon group, a C3-C10 cycloalkyl group, and a C6-C10 aryl group; preferably, R is selected from a C4-C6 branched-chain alkyl and a C4-C6 cycloalkyl; and more preferably, R is 1-methylpropyl, 1,3-dimethylbutyl, or cyclohexyl.

In one or more embodiments, in Formula B, R_{c} and R_{d} are independently selected from a C1-C6 alkyl and a C4-C6 cycloalkyl; and preferably, R_{c} and R_{d} are independently methyl or ethyl.

In one or more embodiments, the compound of Formula A of the present invention has the following structure as shown in Formula I: 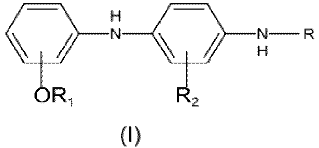
wherein R is selected from a C1-C20 chain hydrocarbon group, a C3-C20 alicyclic hydrocarbon group, a C6-C20 aryl group, and a C1-C20 alkoxy group;
R₁ is selected from a C1-C20 alkyl, a C3-C20 cycloalkyl, and a C7-C20 alkyl phenyl;
R₂ is selected from hydrogen, a C1-C20 alkyl, a C3-C20 cycloalkyl, or a C7-C20 alkyl phenyl.

In one or more embodiments, in Formula I, R is selected from a C3-C10 branched-chain hydrocarbon group, a C3-C10 cycloalkyl group, and a C6-C10 aryl group; preferably, R is selected from a C4-C6 branched-chain alkyl, a C4-C6 cycloalkyl, and phenyl; and more preferably, R is 1-methylpropyl, 1,3-dimethylbutyl, cyclohexyl, or phenyl.

In one or more embodiments, in Formula I, R₁ is selected from a C1-C10 alkyl, a C3-C10 cycloalkyl, and a C7-C10 alkyl phenyl; preferably, R₁ is selected from a C1-C6 alkyl and a C4-C6 cycloalkyl; and more preferably, R₁ is methyl, or ethyl.

In one or more embodiments, in Formula I, R₂ is selected from H, a C1-C10 alkyl, a C3-C10 cycloalkyl, and a C7-C10 alkyl phenyl; preferably, R₂ is selected from H, a C1-C6 alkyl, and a C4-C6 cycloalkyl; and more preferably, R₂ is H, methyl, or ethyl.

In one or more embodiments, the compound of Formula I of the present invention has a structure as shown in Formula II or Formula III: 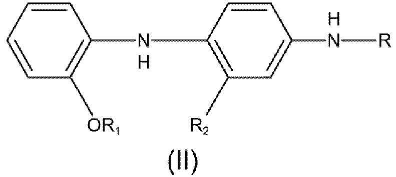 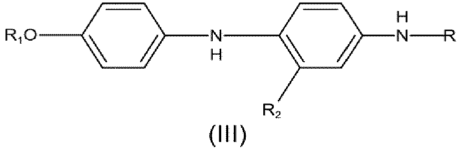 wherein R, R₁, and R₂ in Formula II and Formula III are as defined in any embodiment herein.

In one or more embodiments, the compound of Formula A of the present invention is selected from: 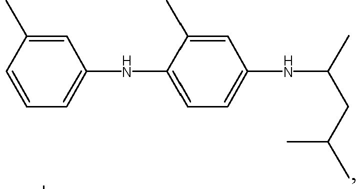 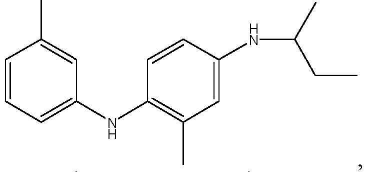 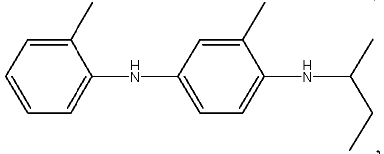 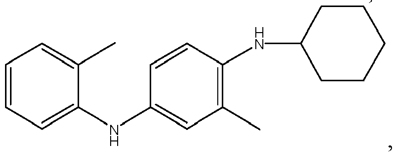 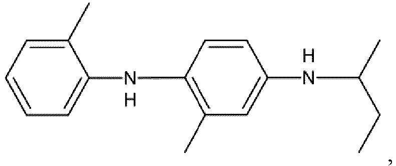 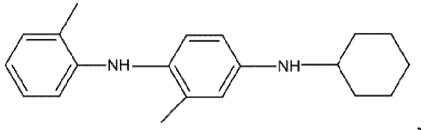 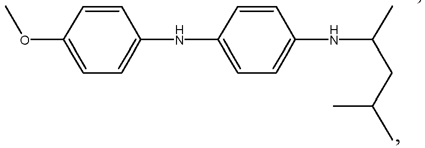 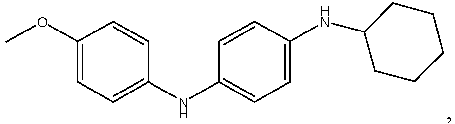 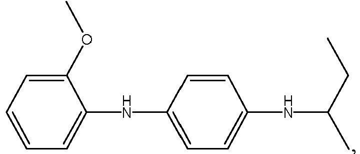 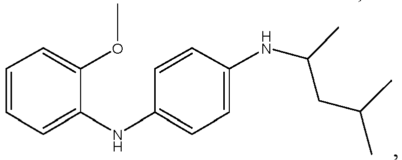 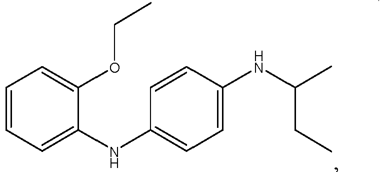 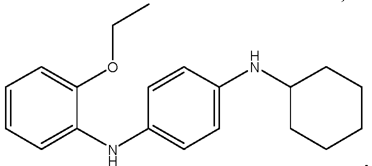 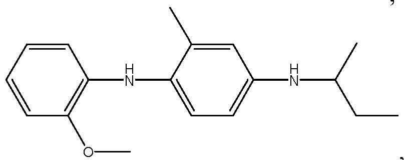 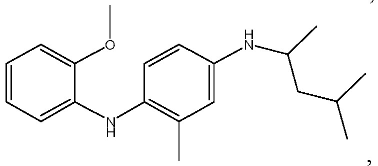 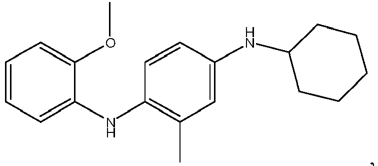 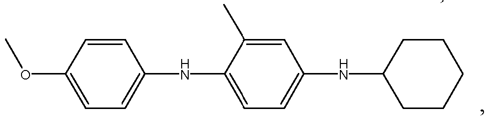 and

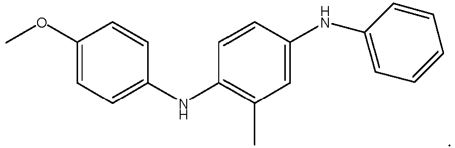

The present invention further provides a method for preparing the compound of Formula A of the present invention, which comprises the following steps:
(1) reacting a compound of Formula C and a compound of Formula D in a condensation reaction in presence of a first catalyst to obtain a condensate comprising a compound of Formula E, a compound of Formula F, or both, and subjecting the condensate to a reduction reaction in presence of H₂ and a second catalyst to obtain a compound of Formula X; 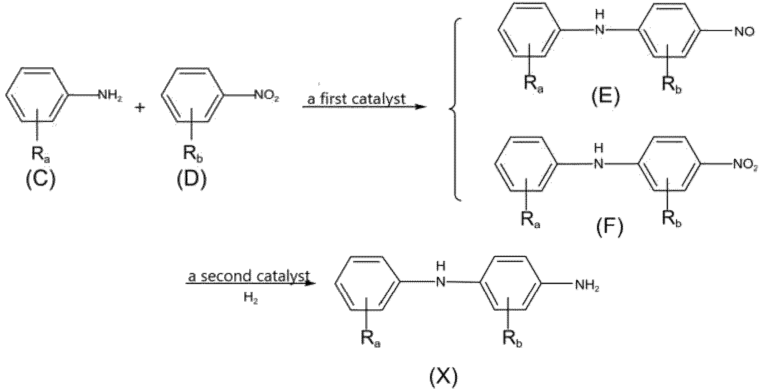
(2) reacting the compound of Formula X with an aldehyde or a ketone in reductive alkylation reaction in presence of H₂ and a third catalyst to obtain the compound of Formula A; 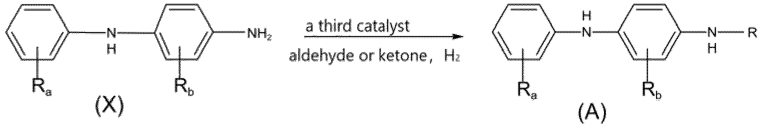 wherein R, Rₐ, R_{b} in Formula C, Formula D, Formula E, Formula F, Formula X, and Formula A are as defined in any embodiment herein.

In one or more embodiments, in step (1), the first catalyst is one or more selected from an alkali metal hydroxide, an alkali metal alkoxide, a quaternary ammonium base, a combination of an alkali metal hydroxide and a halide of tetraalkyl ammonium.

In one or more embodiments, in step (1), the molar ratio of the first catalyst to the compound of Formula C is 0.1: 1 to 2: 1, and preferably 0.9: 1 to 1.1: 1.

In one or more embodiments, in step (1), the molar ratio of the compound of Formula C to the compound of Formula D is 2:1 to 15:1, preferably 4:1 to 10:1, and more preferably 5:1 to 8:1.

In one or more embodiments, the temperature of the condensation reaction of step (1) is 40 to 90°C, and preferably 65 to 85°C.

In one or more embodiments, the condensation reaction of step (1) is carried out under vacuum with a pressure in the range of -0.09 to -0.1MPa.

In one or more embodiments, in step (1), the second catalyst is a porous metal catalyst or a supported metal catalyst. The porous metal catalyst is preferably one or more selected from Raney nickel, Raney cobalt, and Raney copper; the metal in the supported metal catalyst is preferably one or more selected from nickel, cobalt, copper, platinum, palladium, ruthenium, and rhodium; and the support in the supported metal catalyst is preferably one or more selected from carbon, alumina, silica gel, and molecular sieve.

**In** one or more embodiments, in step (1), the mass ratio of the metal in the second catalyst to the condensate is 0.0001: 1 to 0.2: 1.

**In** one or more embodiments, the temperature of the reduction reaction in step (1) is in a range of 40 to 120°C, preferably 60 to 90°C, and the hydrogen pressure is in a range of 0.5 to 5MPa, and preferably 1 to 2MPa.

**In** one or more embodiments, in step (2), the third catalyst is a supported metal catalyst. The metal in the supported metal catalyst is preferably one or more selected from nickel, cobalt, copper, platinum, palladium, ruthenium, and rhodium; and the support in the supported metal catalyst is preferably one or more selected from carbon, alumina, silica gel, and molecular sieve.

**In** one or more embodiments, the molar ratio of the aldehyde or ketone to the compound of Formula X in step (2) is 1: 1 to 15: 1.

**In** one or more embodiments, the temperature of the reductive alkylation reaction in step (2) is in a range of 40 to 150°C, and the hydrogen pressure is in a range of 0.5 to 5MPa.

The present invention further provides a compound of Formula X that may be used as an intermediate for preparing a compound of Formula A of the present invention as follows: 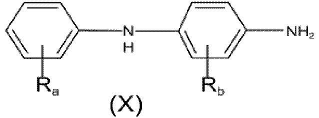 wherein Rₐ and R_{b} in Formula X are as defined in any embodiment herein.

The present invention also provides a method for preparing the compound of Formula X of the present invention, which comprises the steps of reacting the compound of Formula C and the compound of Formula D in the condensation reaction in presence of the first catalyst to obtain the condensate containing the compound of Formula E and/or the compound of Formula F, and then subjecting the condensate to a reduction reaction under the action of H₂ and the second catalyst to obtain the compound of Formula X; 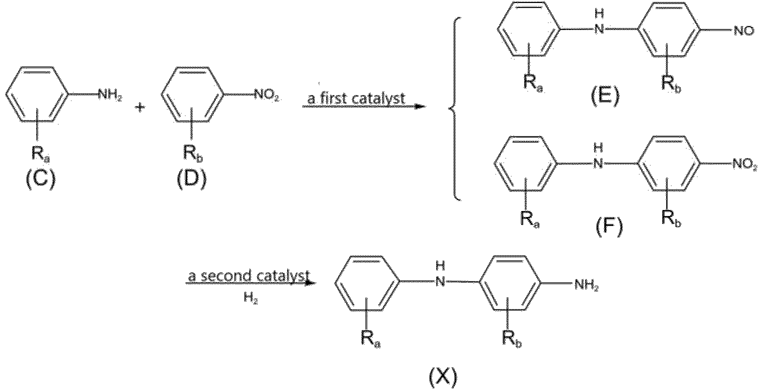 wherein Rₐ and R_{b} in Formulae C, D, E, F, and X are defined in any embodiment herein.

In one or more embodiments, the first catalyst is one or more selected from an alkali metal hydroxide, an alkali metal alkoxide, a quaternary ammonium base, a combination of an alkali metal hydroxide and a halide of tetraalkyl ammonium.

In one or more embodiments, the molar ratio of the first catalyst to the compound of Formula C is 0.1: 1 to 2: 1, preferably 0.1: 1 to 1.1: 1.

In one or more embodiments, the molar ratio of the compound of Formula C to the compound of Formula D is 2: 1 to 15: 1, preferably 4: 1 to 10: 1, and more preferably 5: 1 to 8: 1.

In one or more embodiments, the temperature of the condensation reaction is in a range of 40 to 90°C, preferably 65 to 85°C.

In one or more embodiments, the condensation reaction is carried out under vacuum, and the pressure is in the range of -0.09 to -0.1MPa.

In one or more embodiments, the second catalyst is a porous metal catalyst or a supported metal catalyst. The porous metal catalyst is preferably one or more selected from Raney nickel, Raney cobalt or Raney copper; the metal in the supported metal catalyst is preferably one or more selected from nickel, cobalt, copper, platinum, palladium, ruthenium or rhodium; and the support in the supported metal catalyst is preferably one or more selected from carbon, alumina, silica gel, or molecular sieve.

In one or more embodiments, the mass ratio of the metal in the second catalyst to the condensate is 0.0001: 1 to 0.2: 1.

In one or more embodiments, the temperature of the reduction reaction is in a range of 40 to 120°C, preferably 60 to 90°C.

In one or more embodiments, the hydrogen pressure in the reduction reaction is in a range of 0.5 to 5MPa, preferably 1 to 2 MPa.

The present invention further provides a rubber composition, which comprises a compound of Formula A, a compound of Formula B, a compound of Formula I, and/or a compound of Formula II of the present invention.

The present invention further provides a rubber product comprising the rubber composition of the present invention. Preferably, the rubber product is a tire.

The present invention also provides a method for improving thermal oxidative aging resistance, ozone aging resistance, and/or discoloration resistance of rubber or rubber products, wherein the method comprises adding a compound of Formula A, a compound of Formula B, a compound of Formula I, and/or a compound of Formula II of the present application to rubber or rubber products.

### BRIEF DESCRIPTION OF THE DRAWING

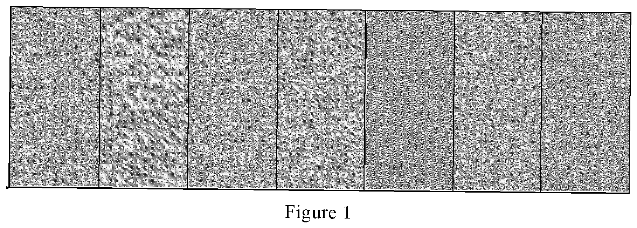

Figure 1 shows the simulated color of each rubber material aging for 15 days in the test example, among which, the simulated color from left to right are of rubber material 1, rubber material 2, rubber material 3, rubber material 4, rubber material 5, rubber material 6 and rubber material 7.

### DETAILED DESCRIPTION OF THE INVENTION

In order to enable those skilled in the art to understand the characteristics and effects of the present invention, the terms and expressions mentioned in the description and claims are generally described and defined below. Unless otherwise specified, all technical and scientific terms used in the text have their usual meanings as understood by those skilled in the art regarding the present invention. In the event of conflict, the definitions in this specification shall prevail.

The theories or mechanisms described and disclosed herein, whether true or false, should not limit the scope of the invention in any way, that is, the invention may be implemented without being limited to any specific theory or mechanism.

In the present invention, all characteristics such as numerical values, amounts, contents, and concentrations defined in the form of numerical ranges or percentage ranges are for brevity and convenience only. Accordingly, descriptions of numerical ranges or percentage ranges shall be deemed to cover and specifically disclose all possible subranges and individual values within the ranges (including integers and fractions).

In the present invention, for the sake of conciseness, not all possible combinations of each technical feature in each embodiment or example are described. Thus, as long as there is no contradiction in the combination of these technical features, each technical feature in each embodiment or example may be arbitrarily combined, and all possible combinations should be considered to be within the scope of the specification.

In the present invention, a chain hydrocarbon group refers to a linear or branched saturated hydrocarbon group or unsaturated hydrocarbon group, usually containing 1-20 carbon atoms (a C1-C20 chain hydrocarbon group), for example, containing 1-10 carbon atoms (a C1-C10 chain hydrocarbon group). Examples of a chain hydrocarbon group include, but are not limited to, methyl, ethyl, n-propyl, isopropyl, n-butyl, 1-methylpropyl, sec-butyl, tert-butyl, n-hexyl, isohexyl, 1,3-dimethylbutyl, 1,4-dimethylpentyl, tert-octyl, vinyl, propenyl, and ethynyl.

In the present invention, an alicyclic hydrocarbon group refers to a group of carbon atoms bound in a cyclic form, usually containing 3-20 carbon atoms (a C3-C20 alicyclic hydrocarbon group), for examples, containing 3-10 carbon atoms (a C3-C10 alicyclic hydrocarbon group). Examples of an alicyclic hydrocarbon group include, but are not limited to, isobornyl, cyclohexyl, norbornanyl, norbornenyl, dicyclopentadienyl, ethynyl cyclohexanyl, and ethynyl cyclohexenyl.

In the present invention, an aryl group refers to a monovalent group derived from removing a hydrogen atom from a carbon atom on the aromatic ring (ring-carbon atom) of an aromatic molecule. The number of ring-carbon atoms of aryl is usually 6 to 20. Examples of aryl groups include phenyl and naphthyl. The aryl group may optionally be substituted by an alkyl, cycloalkyl, aryl, or a combination thereof. The number of substituents is usually 1 or 2.

In the present invention, an alkyl group refers to a linear or branched monovalent saturated hydrocarbon group, usually containing 1 to 20 carbon atoms (C1 to C20 alkyl), for examples, containing 1 to 10 carbon atoms (C1 to C10 alkyl). Examples of the alkyl groups include, but are not limited to, methyl, ethyl, propyl, 1-methylpropyl, and 1,3-dimethylbutyl.

In the present invention, an alkoxy group refers to a combination of an alkyl group and an oxygen atom, which may contain 1 to 20 carbon atoms (C1 to C20 alkoxy). Alkoxy groups can be classified as straight-chain, branched-chain, or cyclic structures. Examples of alkoxy groups include, but are not limited to, methoxy, ethoxy, n-propoxy, and isopropoxy.

In the present invention, a cycloalkyl group refers to a monovalent saturated hydrocarbon ring containing 3-10 carbon atoms, preferably containing 3-8 carbon atoms. Examples of cycloalkyl groups include, but are not limited to, cyclopropyl, cyclobutyl, cyclopentyl, cyclohexyl, cycloheptyl, cyclooctyl, and adamantyl.

In the present invention, alkyl phenyl refers to phenyl substituted with one or more alkyl groups, with a total of carbon atoms (including phenyl) usually not exceeding 20, preferably containing 7 to 10 carbon atoms (C7 to C10 alkyl phenyl). Examples of alkyl phenyls include, but are not limited to, tolyl, ethylphenyl, propylphenyl, and butylphenyl.

### Compound of Formula A

It is found in the present invention that a compound having the structure represented by Formula A (compound of Formula A) can be used as a rubber antidegradant and has a close or better anti-aging performance, discoloration resistance, and/or durable performance compared with the antidegradant 6PPD, and has little effect on the processing/vulcanization properties of rubber and physical properties of rubber before aging: 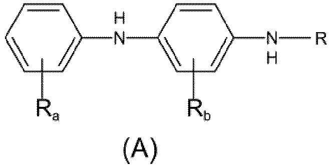
wherein R is selected from C1-C20 chain hydrocarbon group, C3-C20 alicyclic hydrocarbon group, C6-C20 aryl group, and C1-C20 alkoxy group;
Rₐ is selected from H, C1-C20 alkyl, C3-C20 cycloalkyl, phenyl, C7-C20 alkyl phenyl, C1-C20 alkyloxy, C3-C20 cycloalkyloxy, and C7-C20 alkylphenyloxy;
R_{b} is selected from H, C1-C20 alkyl, C3-C20 cycloalkyl, phenyl, and C7-C20 alkyl phenyl;
and the compounds of Formula A do not include compounds where Rₐ is selected from H, C1-C20 alkyl, C3-C20 cycloalkyl, phenyl, and C7-C20 alkyl phenyl and R_{b} is H.

In some embodiments, in Formula A, Rₐ is selected from C1-C20 alkyl, C3-C20 cycloalkyl, phenyl, C7-C20 alkyl phenyl, C1-C20 alkyloxy, C3-C20 cycloalkyloxy, and C7-C20 alkylphenyloxy.

In some embodiments, in Formula A, Rₐ is selected from C1-C10 alkyl, C3-C10 cycloalkyl, phenyl, C7-C10 alkyl phenyl, C3-C10 branched alkyloxy, C3-C10 cycloalkyloxy, and C7-C10 alkylphenyloxy.

In some embodiments, in Formula A, R_{b} is selected from the group consisting of H, C1-C10 alkyl, C3-C10 cycloalkyl, phenyl and C7-C10 alkyl phenyl.

### Compound of Formula B

In some embodiments, the compound of Formula A of the present invention has a structure represented by Formula B: 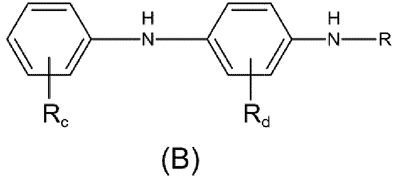
wherein R is selected from a C1-C20 chain hydrocarbon group, a C3-C20 alicyclic hydrocarbon group, and a C6-C20 aryl group;
R_{c} and R_{d} are independently selected from a C1-C10 alkyl, a C3-C10 cycloalkyl, phenyl, and a C7-C10 alkyl phenyl.

In some embodiments, in Formula B, R is selected from a C1-C8 chain hydrocarbon group, a C3-C18 alicyclic hydrocarbon group, and a C6-C18 aryl group.

In some embodiments, in Formula B, R is selected from a C3-C10 branched hydrocarbon group (for example, C3-C10 branched alkyl), a C3-C10 cycloalkyl, and a C6-C10 aryl. Examples of a C3-C10 branched alkyl group include isopropyl, 1-methylpropyl, 1-methylbutyl, 1,2-dimethylpropyl, 1-methylpentyl, 1,2-dimethylbutyl, 1,3-dimethylbutyl, 1-ethylbutyl, 2-heptyl, 1,2-dimethylpentyl, 1,3-dimethylpentyl, 1,4-dimethylpentyl, 3-heptyl, 4-heptyl, 2-octyl, 3-octyl, 4-octyl, 1,2-dimethylhexyl, 1,3-dimethylhexyl, and 1,4-dimethylhexyl. Examples of C3-C10 cycloalkyl groups include cyclohexyl. In some preferred embodiments, in Formula B, R is selected from a C4-C6 branched alkyl and a C4-C6 cycloalkyl, more preferably R is 1-methylpropyl, 1,3-dimethylbutyl, or cyclohexyl.

In Formula B, R_{c} and R_{d} may be the same or different. In some embodiments, in Formula B, R_{c} and R_{d} are independently selected from a C1-C6 alkyl and a C4-C6 cycloalkyl. In some preferred embodiments, in Formula B, R_{c} and R_{d} are independent methyl or ethyl. In some embodiments, R_{c} is methyl or ethyl, and R_{d} is methyl. In some embodiments, R_{c} and R_{d} are methyl.

In Formula B, the position of R_{c} and R_{d} on the benzene ring is not particularly limited.

In some embodiments, in Formula B, R_{c} is at the meta-position of the -NH- group and R_{d} is at the meta-position of the -NH-R group. In some embodiments, in Formula B, R_{c} is at the ortho-position of the -NH- group and R_{d} is at the ortho-position of the -NH-R group. In some embodiments, in Formula B, R_{c} is at the ortho-position of the -NH- group and R_{d} is at the meta-position of the -NH-R group. In some embodiments, in Formula B, R_{c} is at the meta-position of the -NH- group and R_{d} is at the ortho-position of the -NH-R group.

In some embodiments, the compound of Formula B is selected from: 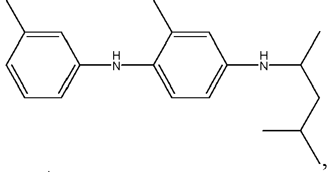 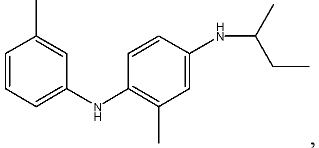 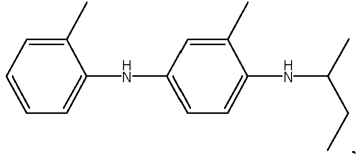 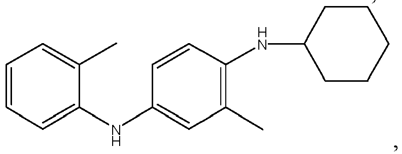 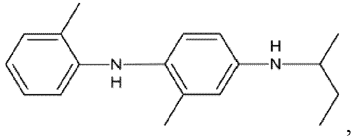 and

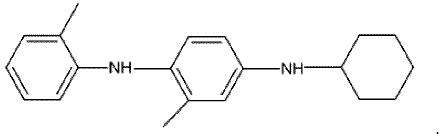

### Compound of Formula B'

In some embodiments, the compound of Formula A of the present invention has a structure represented by Formula B': 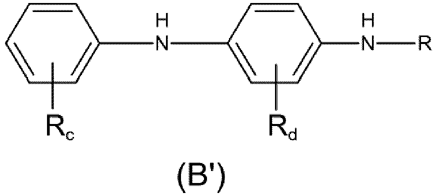
wherein R is selected from a C1-C20 chain hydrocarbon group, a C3-C20 alicyclic hydrocarbon group, and a C6-C20 aryl group;
R_{c} is H;
R_{d} is selected from a C1-C10 alkyl, a C3-C10 cycloalkyl, phenyl, and a C7-C10 alkyl phenyl.

In some embodiments, in Formula B', R is selected from a C1-C18 chain hydrocarbon group, a C3-C18 alicyclic hydrocarbon group, and a C6-C18 aryl group.

In some embodiments, in Formula B', R is selected from a C3-C10 branched hydrocarbon group (for example, a C3-C10 branched alkyl), a C3-C10 cycloalkyl, and a C6-C10 aryl. Examples of C3-C10 branched alkyl groups include isopropyl, 1-methylpropyl, 1-methylbutyl, 1,2-dimethylpropyl, 1-methylpentyl, 1,2-dimethylbutyl, 1,3-dimethylbutyl, 1-ethylbutyl, 2-heptyl, 1,2-dimethylpentyl, 1,3-dimethylpentyl, 1,4-dimethylpentyl, 3-heptyl, 4-heptyl, 2-octyl, 3-octyl, 4-octyl, 1,2-dimethylhexyl, 1,3-dimethylhexyl, and 1,4-dimethylhexyl. Examples of C3-C10 cycloalkyl groups include cyclohexyl. In some preferred embodiments, in Formula B', R is selected from a C4-C6 branched alkyl, a C4-C6 cycloalkyl, and a C6-C10 aryl, more preferably R is 1-methylpropyl, 1,3-dimethylbutyl, cyclohexyl, or phenyl.

In Formula B', the position of R_{d} on the benzene ring is not particularly limited.

In some embodiments, in Formula B', R_{d} is at the meta-position of the -NH-R group. In some embodiments, in Formula B', R_{d} is at the ortho-position of the -NH-R group.

In some embodiments, the compound of Formula B' is selected from: 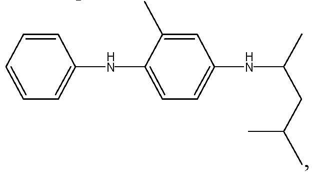 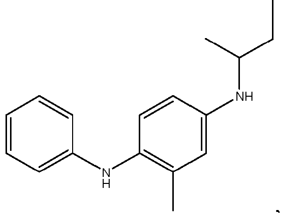 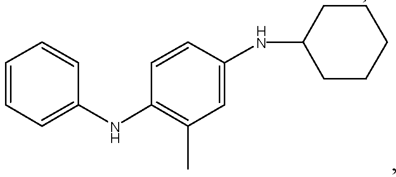 and

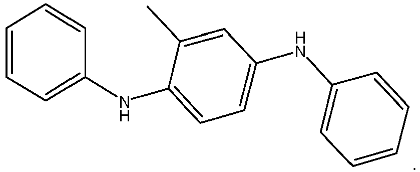

### Compound of Formula I

In some embodiments, the compound of Formula A of the present invention has the structure represented by Formula I. The compound having the structure represented by Formula I (compound of Formula I) of the present invention can be used as a rubber antidegradant and has a close or better ozone aging resistance, thermal oxidative aging resistance, discoloration resistance, and durable performance compared with the antidegradant 6PPD, and has little effect on the processing/vulcanization properties of rubber and physical properties of rubber before aging: 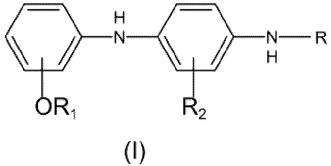
wherein R is selected from a C1-C20 chain hydrocarbon group, a C3-C20 alicyclic hydrocarbon group, a C6-C20 aryl group, and a C1-C20 alkoxy group;
R₁ is selected from a C1-C20 alkyl, a C3-C20 cycloalkyl, and a C7-C20 alkyl phenyl;
R₂ is selected from H, a C1-C20 alkyl, a C3-C20 cycloalkyl, and a C7-C10 alkyl phenyl.

In a preferred embodiment, R is selected from a C3-C10 branched-chain hydrocarbon group, a C3-C10 cycloalkyl group, and a C6-C10 aryl group. Examples of C3-C10 branched-chain alkyl groups include isopropyl, 1-methylpropyl, 1-methylbutyl, 1,2-dimethylpropyl, 1-methylpentyl, 1,2-dimethylbutyl, 1,3-dimethylbutyl, 1-ethylbutyl, 2-heptyl, 1,2-dimethylpentyl, 1,3-dimethylpentyl, 1,4-dimethylpentyl, 3-heptyl, 4-heptyl, 2-octyl, 3-octyl, 4-octyl, 1,2-dimethylhexyl, 1,3-dimethylhexyl, and 1,4-dimethylhexyl. Examples of C3-C10 cycloalkyl groups include cyclopentyl, cyclohexyl, cycloheptyl, or cyclooctyl. Examples of C6-C10 aryl groups include phenyl, tolyl, ethylphenyl, xylene, and naphthyl. In some embodiments, R is selected from a C4-C6 branched-chain alkyl, a C4-C6 cycloalkyl, and phenyl. In some other embodiments, R is 1-methylpropyl, 1,3-dimethylbutyl, cyclohexyl, or phenyl.

In a preferred embodiment, R₁ is selected from a C1-C10 alkyl, a C3-C10 cycloalkyl, and a C7-C10 alkyl phenyl. In some embodiments, R₁ is selected from a C1-C10 alkyl group and a C3-C10 cycloalkyl group. In some embodiments, R₁ is selected from a C1-C6 alkyl and a C4-C6 cycloalkyl. In some embodiments, R₁ is selected from a C1-C6 alkyl. In some embodiments, R₁ is methyl or ethyl.

In some embodiments, in the compound of Formula I, R₁O- group is at the ortho-or para-position of the -NH- group on the benzene ring where it locates.

In a preferred embodiment, R₂ is selected from H, a C1-C10 alkyl, a C3-C10 cycloalkyl, and a C7-C10 alkyl phenyl. In some embodiments, R₂ is selected from H, a C1-C10 alkyl, and a C3-C10 cycloalkyl. In some embodiments, R₂ is selected from H, a C1-C6 alkyl, and a C4-C6 cycloalkyl. In some embodiments, R₂ is selected from H, a C1-C6 alkyl, and a C4-C6 cycloalkyl. In some embodiments, R₂ is H, methyl, or ethyl.

In some embodiments, in the compound of Formula I, R₁O- group is at the meta-position of the -NHR group on the benzene ring where it locates.

In some embodiments, R₂ is H; R is selected from a C3-C10 branched-chain hydrocarbon group and a C3-C10 cycloalkyl, preferably a C4-C6 branched-chain alkyl group and a C4-C6 cycloalkyl; R₁ is selected from a C1-C10 alkyl, a C3-C10 cycloalkyl, and a C7-C10 alkyl phenyl, preferably a C1-C10 alkyl and a C3-C10 cycloalkyl, and more preferably a C1-C6 alkyl and C4-C6 cycloalkyl, such as C1-C6 alkyl. In some embodiments, R₂ is H, R is 1-methylpropyl, 1,3-dimethylbutyl, or cyclohexyl, and R₁ is methyl or ethyl.

In some embodiments, R₂ is selected from a C1-C10 alkyl, a C3-C10 cycloalkyl, and a C7-C10 alkyl phenyl, preferably a C1-C10 alkyl and a C3-C10 cycloalkyl, more preferably a C1-C6 alkyl and a C4-C6 cycloalkyl, such as C1-C6 alkyl; R is selected from a C3-C10 branched-chain hydrocarbon group, a C3-C10 cycloalkyl, and a C6-C10 aryl, preferably a C4-C6 branched-chain alkyl, a C4-C6 cycloalkyl, and phenyl; R₁ is selected from a C1-C10 alkyl, a C3-C10 cycloalkyl, and a C7-C10 alkyl phenyl, preferably a C1-C10 alkyl and a C3-C10 cycloalkyl, more preferably a C1-C6 alkyl and a C4-C6 cycloalkyl, such as a C1-C6 alkyl. In some embodiments, R₂ is methyl or ethyl, R is 1-methylpropyl, 1,3-dimethylbutyl, cyclohexyl, or phenyl, and R₁ is methyl or ethyl.

In some embodiments, the compound of Formula I of the present invention has a structure represented by Formula II or Formula III: 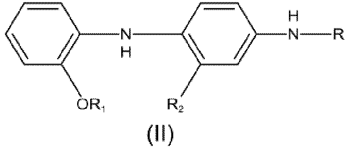 or 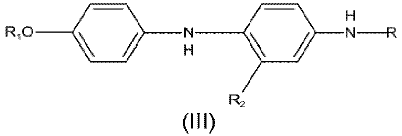 wherein R, R₁ and R₂ in Formula II and Formula III are as defined above.

It is found in the present invention that the compound of Formula II of the present invention can impart rubber better thermal oxidative aging resistance than the antidegradant 6PPD.

In some embodiments, the compound of Formula I of the present invention is selected from: 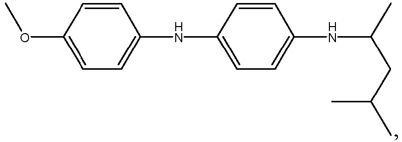 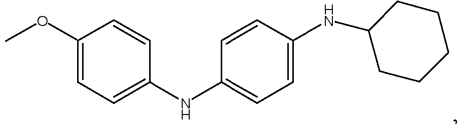 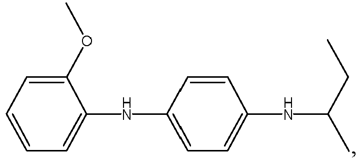 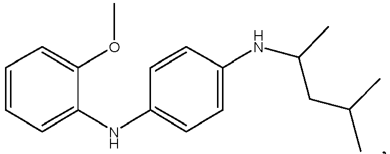 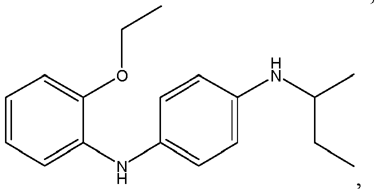 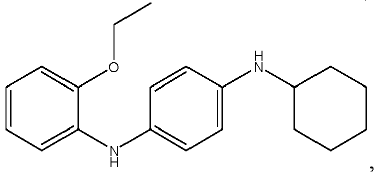 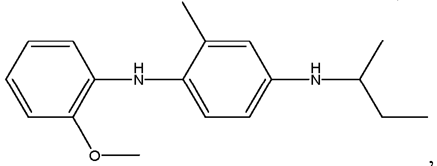 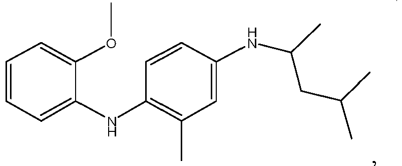 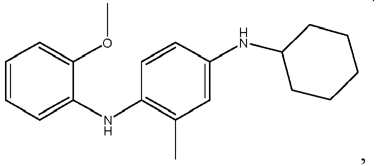 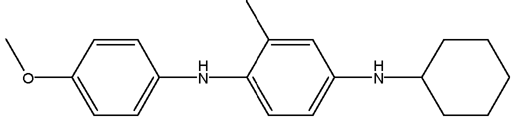 and

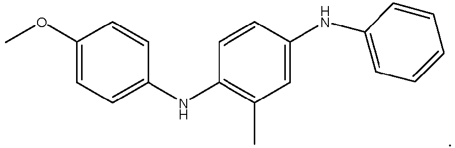

### Compound of Formula X

The present invention also provides a compound of Formula X that may be used as an intermediate to prepare a compound of Formula A, a compound of Formula B, a compound of Formula B', a compound of Formula I, a compound of Formula II, and a compound of Formula III: 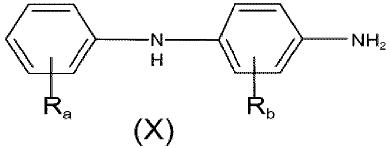 wherein in Formula X, Rₐ and R_{b} are as defined by Rₐ and R_{b} in any preceding embodiments of the compound of Formula A, or as defined by R_{c} and R_{d} in any preceding embodiments of the compound of Formula B or B', or as defined by -OR₁ and R₂ in any preceding embodiments of the compound of Formula I, the compound of Formula II, or the compound of Formula III, respectively.

In some embodiments, as an intermediate of the aforementioned compounds of Formula II and Formula III, the compound of Formula X according to the present invention has a structure represented by Formula XI or Formula XII: 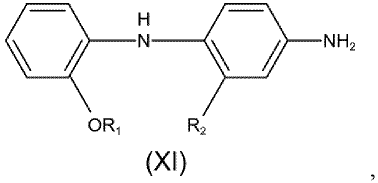 or 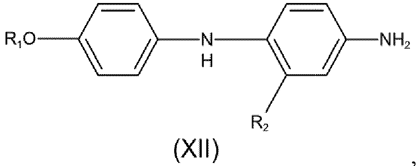 wherein R₁ and R₂ in Formula XI and Formula XII are as defined in any preceding embodiments of the compound of Formula I.

In some embodiments, the compound of Formula X is selected from: 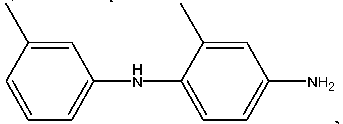 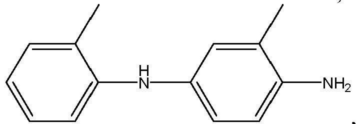 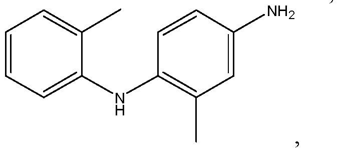 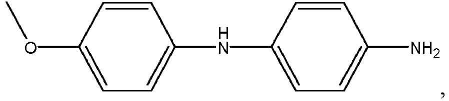 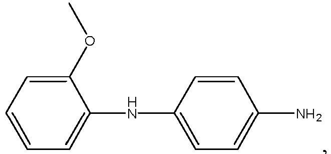 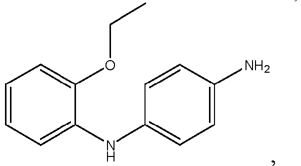 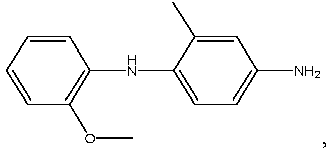 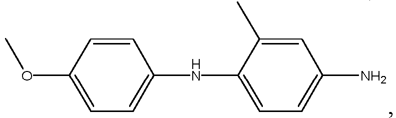 and

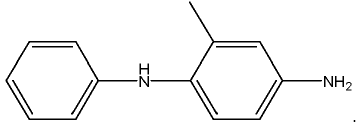

### Preparation method for compound of Formula A and compound of Formula X

The method for preparing the compound of Formula X and the compound of Formula A according to the present invention comprises the following steps:
(1) subjecting a compound of Formula C and a compound of Formula D to a condensation reaction in presence of a first catalyst to obtain a condensate containing a compound of Formula E and/or a compound of Formula F, and then subjecting the condensate to a reduction reaction in presence of H₂ and a second catalyst to obtain the compound of Formula X; 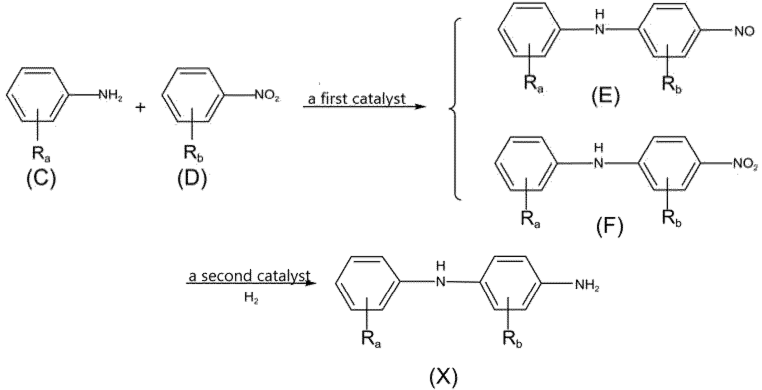
(2) reacting the compound of Formula X with an aldehyde or a ketone through reductive alkylation reaction in presence of H₂ and a third catalyst to obtain the compound of Formula A; 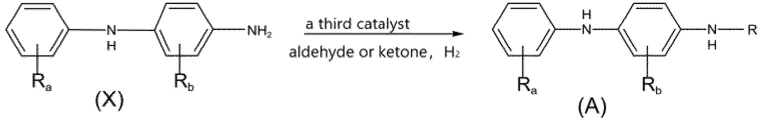 wherein R, Rₐ, R_{b} in Formula C, Formula D, Formula E, Formula F, Formula X and Formula A are as defined in any of the embodiments herein.

The corresponding Rₐ and R_{b} groups in Formula C and Formula D may be determined according to the Rₐ and R_{b} groups contained in the compound of Formula A according to the present invention, or the R_{c} and R_{d} groups contained in the compound of Formula B or B' according to the present invention, or the -OR₁ and R₂ groups contained in the compound of Formula I, the compound of Formula II or the compound of Formula III according to the present invention. Suitable aldehyde or ketone in step (2) may be determined according to the R group contained in the compound of Formula A, the compound of Formula B, the compound of Formula B', the compound of Formula I, the compound of Formula II or the compound of Formula III. Thus, a compound of Formula A, a compound of Formula B, a compound of Formula B', a compound of Formula I, a compound of Formula II or a compound of Formula III are prepared accordingly.

The first catalyst used in step (1) may be one or more selected from an alkali metal hydroxide, an alkali metal alkoxide, a quaternary ammonium base, or a combination of an alkali metal hydroxide and a halide of tetraalkyl ammonium. Alkali metal hydroxides suitable for the present invention include sodium hydroxide, potassium hydroxide, lithium hydroxide, etc. Alkali metal alkoxides suitable for the present invention include sodium methoxide, potassium methoxide, sodium ethoxide, potassium ethoxide, sodium tert-butoxide, potassium tert-butoxide, sodium tert-pentoxide, potassium tert-pentoxide, etc. Quaternary ammonium bases are compounds having the general formula of R₄NOH, where R is four identical or different aliphatic hydrocarbon groups or aromatic hydrocarbon groups. The R group in the quaternary ammonium base suitable for the present invention may be one or more selected from methyl, ethyl, propyl, butyl, etc. Examples of quaternary ammonium bases suitable for the present invention include tetramethylammonium hydroxide, tetraethylammonium hydroxide, tetrabutylammonium hydroxide, etc. The first catalyst can also be a combination of alkali metal hydroxides and halides of tetraalkyl ammonium. Chloride of tetraalkyl ammonium has the general formula of R₄NX, wherein R is four identical or different aliphatic hydrocarbon groups or aromatic hydrocarbon groups, such as methyl, ethyl, propyl, or butyl, etc., and X is a halogen atom, such as fluorine, chlorine, bromine, or iodine. Examples of the combination of alkali metal hydroxides and halides of tetraalkyl ammonium include sodium hydroxide and tetrabutylammonium bromide and the like. The molar ratio of the first catalyst to the compound of Formula C may be 0.1: 1 to 2: 1, preferably 0.9: 1 to 1.1: 1, such as 1.05:1, 1.1:1, or 1.5:1.

In some embodiments, in step (1), the compound of Formula C firstly reacts with the first catalyst to form a salt, and then, the compound of Formula D is added dropwise to carry out the condensation reaction.

In step (1), the condensate obtained by the condensation reaction of the compounds of Formula C and Formula D in the presence of the first catalyst may be one or both of the nitro-compounds represented by Formula E and the nitroso-compounds represented by Formula F, and may also contain azobenzene compounds. The molar ratio of the compound of Formula C to the compound of Formula D may be 2:1 to 15:1, preferably 4:1 to 10:1, and more preferably 5:1 to 8:1, such as 6:1 or 7:1.

In step (1), the condensation reaction may be carried out at 40 to 90°C, preferably 65 to 85°C, for example, the reaction temperature may be 60°C, 70°C, 75°C, or 80°C. The condensation reaction needs to be carried out under vacuum with a pressure in the range of - 0.09 to -0.1MPa.

The second catalyst used in step (1) may be a porous metal catalyst or a supported metal catalyst. Porous metal catalysts are also known as sponge metal catalysts. Porous metal catalysts suitable for the present invention include Rainey nickel (also known as skeleton nickel), Rainey cobalt, Rainey copper, and the like. Supported metal catalysts include metals that act as centers of catalytic activity and supports that are used for supporting metals. The metal in the supported metal catalyst suitable for the present invention may be nickel, cobalt, copper, platinum, palladium, ruthenium, rhodium, etc. The support may be carbon, alumina, silica gel, molecular sieve, etc. The carbon as a support may be activated carbon. The molar ratio of the metal in the second catalyst to the condensate may be 0.0001:1 to 0.2:1.

In step (1), the condensate generated by the condensation reaction is subjected to a hydrogenation reduction reaction in the presence of a second catalyst to generate the compound of Formula X. In step (1), the reduction reaction may be carried out at 40 to 120°C, preferably 60 to 90°C, for example, the reaction temperature may be 70°C, 75°C, or 80°C. The hydrogen pressure in the reduction reaction may be 0.5 to 5MPa, such as 1MPa, 1.5MPa, 2MPa, or 2.5MPa.

In step (1), Compound C itself may be used as a solvent, or solvents such as toluene and xylene may also be used. At the end of the reaction in step (1), after the reaction liquid is filtered, washed with water, and separated, the organic phase is distilled under reduced pressure to remove the light components to obtain the compound of Formula X.

The third catalyst used in step (2) may be the aforementioned supported metal catalyst, such as Pt/C. The molar ratio of the metal in the third catalyst to the compound of Formula X may be 0.0001:1 to 0.2:1.

In step (2), the compound of Formula X and aldehyde or ketone undergo hydroreductive alkylation reaction in the presence of a third catalyst to generate the compound of Formula A. After the reaction, the carbon atom of carbonyl in the aldehyde or ketone is linked to the nitrogen atom of amino in the compound of Formula A. Therefore, the appropriate aldehyde or ketone can be selected for the reaction according to the R group contained in the compound of Formula A to be prepared, for example, the compound of Formula A with an R group of 1,3-dimethylbutyl may be prepared using 4-methyl-2-pentanone, and the compound of Formula A with an R group of cyclohexyl may be prepared using cyclohexanone. When the R group in the compound of Formula A is aryl, ketones as aryl precursors, hydrogen acceptors and water-carrying agents are added to the reaction system for reaction. For example, cyclohexanone, hydrogen acceptors, and water-carrying agents may be used to prepare the compound of Formula A with R being phenyl. The hydrogen acceptor may be nitrobenzene. The water-carrying agent may be toluene. The molar ratio of aldehydes or ketones to the compound of Formula X may be 1:1 to 15:1, such as 2:1, 3:1, 5:1, 8:1, or 10:1. The reaction temperature of step (2) may be in the range of 40 to 150°C, such as 50°C, 80°C, 100°C, or 120°C. The hydrogen pressure in step (2) may be 0.5 to 5MPa, such as 1MPa, 1.5MPa, 2MPa, or 2.5MPa.

In step (2), the raw materials aldehydes or ketones for reaction may be used as solvents. At the end of the reaction in step (2), the reaction liquid is filtered and distilled under reduced pressure to remove the light components to obtain the compound of Formula A.

In the present invention, liquid chromatography (LC) or gas chromatography (GC) may be used to determine whether each step of the reaction reaches the endpoint, thereby determining the appropriate reaction time.

The method for preparing the compound of Formula X and the compound of Formula A according to the present invention is green and environmentally friendly, with substantially no wastewater. Since there is no need to use expensive bromide as raw materials and the catalysts can be recycled, it has the advantages of less solid waste and low reaction temperature.

### Rubber compositions and rubber products

The present invention also provides a rubber composition, which comprises the compound of Formula A, the compound of Formula B, the compound of Formula B', the compound of Formula I, the compound of Formula II, or the compound of Formula III according to the present invention as an antidegradant. Hereinafter, the compound of Formula A, the compound of Formula B, the compound of Formula B', the compound of Formula I, the compound of Formula II, and the compound of Formula III are referred to as an antidegradant of the present invention.

The raw materials of the rubber composition typically comprise diene elastomers, reinforcing fillers, antidegradants, and cross-linking agents. In the present invention, the rubber composition comprises unvulcanized rubber and vulcanized rubber. Vulcanized rubber may be prepared by vulcanizing (curing) unvulcanized rubber.

The raw materials of the rubber composition of the present invention comprises 100 parts by weight of a diene elastomer, 30 to 70 parts by weight of a reinforcing filler, 0.1 to 8 parts by weight of an antidegradant, and 0.5 to 3 parts by weight of a crosslinking agent. In the present invention, unless otherwise specified, the part by weight is based on 100 parts by weight of the diene elastomer contained in the raw material of the rubber composition.

In the present invention, diene elastomers refer to elastomers whose monomers comprise diolefins (such as butadiene, isoprene). Diene elastomers suitable for the present invention may be various diene elastomers known in the art, including, but not limited to, one or more of natural rubber (NR), cis-butadiene rubber (BR), isoprene rubber, styrenebutadiene rubber (SBR), chloroprene rubber (CR), nitrile rubber (NBR), isoprene/butadiene copolymer, isoprene/styrene copolymer and isoprene/butadiene/styrene copolymer. In some embodiments, in the raw material of the rubber composition of the present invention, the diene elastomer comprises natural rubber and cis-butadiene rubber, or consists of natural rubber and cis-butadiene rubber. The mass ratio of natural rubber and cis-butadiene rubber may be 1:9 to 9:1, 2:8 to 8:2, 3:7 to 7:3, 4:6 to 6:4, 4.5:5.5 to 5.5:4.5, or 1:1.

The raw materials of the rubber composition of the present invention typically comprise 0.1 to 8 parts by weight, preferably 1 to 5 parts by weight, and more preferably 2±0.5 parts by weight of the antidegradant. The rubber composition of the present invention is characterized in that the antidegradant comprises an antidegradant of the present invention. In the present invention, the antidegradant of the present invention may account for more than 50%, more than 60%, more than 80%, more than 90% or 100% of the total mass of the antidegradant contained in the rubber composition.

The reinforcing filler suitable for the present invention may be a reinforcing filler conventionally used in a rubber composition, including, but not limited to, one or more selected from carbon black, titanium oxide, magnesium oxide, calcium carbonate, magnesium carbonate, aluminum hydroxide, magnesium hydroxide, clay and talc. In some embodiments, in the rubber composition of the present invention, the reinforcing filler is carbon black. The raw material of the rubber composition typically comprises 30 to 70 parts by weight, preferably 40-60 parts by weight, and more preferably 45 to 55 parts by weight of the reinforced filler. In some embodiments, the raw material of the rubber composition of the present invention comprises 30-70 parts by weight, preferably 40 to 60 parts by weight, more preferably 45 to 55 parts by weight, such as 50±2 parts by weight of carbon black.

The crosslinking agent may be sulfur. The raw material of the rubber composition usually comprises 0.5 to 3 parts by weight, preferably 1 to 3 parts by weight, and more preferably 1 to 2 parts by weight of the crosslinking agent. In some embodiments, the raw material of the rubber composition of the present invention comprises 0.5 to 3 parts by weight, preferably 1 to 3 parts by weight, more preferably 1 to 2 parts by weight, such as 1.5±0.2 parts by weight, 1.5± 0.1 parts by weight of the crosslinking agent, such as sulfur.

The raw materials of the rubber composition of the present invention may also comprise other components commonly used in rubber compositions, including but not limited to one or more additives and accelerators. The respective amounts of additives and accelerators may be a routine amount in the art.

Additives may comprise softeners used to improve properties such as processability. Softeners may include petroleum-based softeners (operating oils) such as naphthenic oils, aromatic oils, processing oils, lubricating oils, paraffins, liquid paraffins, petroleum bitumen, and vaseline, and may also include fatty oil-based softeners such as stearic acid, castor oil, flaxseed oil, rapeseed oil, coconut oil, waxes (such as beeswax, carnauba wax and lanolin), tall oil, linoleic acid, palmitic acid, and lauric acid, etc. Additives may also include active agents, such as zinc oxide, which can accelerate the vulcanization rate, improve thermal conductivity, wear resistance, tear resistance, etc. of rubber. Typically, a total of 2 to 20 parts by weight of additives is used per 100 parts by weight of diene elastomer. In some embodiments, the raw material of the rubber composition of the present invention comprises an operating oil, such as aromatic oil. The raw materials of the rubber composition of the present invention may comprise 0 to 20 parts by weight, preferably 1 to 10 parts by weight, more preferably 2 to 8 parts by weight, such as 5±2 parts by weight, 5±1 parts by weight of the operating oil, such as aromatic oil. In some embodiments, the raw materials of the rubber composition of the present invention comprise fatty oil-based softeners, such as stearic acid. The raw material of the rubber composition of the present invention may comprise 0 to 5 parts by weight, preferably 0.5 to 4 parts by weight, more preferably 1 to 3 parts by weight, such as 2±0.5 parts by weight, 2±0.2 parts by weight of fatty oil-based softener, such as stearic acid. In some embodiments, the raw material of the rubber composition of the present invention comprises an active agent, such as zinc oxide. The raw material of the rubber composition of the present invention may comprise 0 to 10 parts by weight, preferably 2 to 8 parts by weight, more preferably 3 to 7 parts by weight, such as 5±1 parts by weight of the active agent, such as zinc oxide. In some embodiments, the raw materials of the rubber composition of the present invention comprise operating oils, fatty oil-based softeners, and active agents. The respective amount of operating oils, fatty oil-based softeners, and active agents may be as described above.

The accelerator is usually a vulcanization accelerator, which can be one or more of sulfonamide vulcanization accelerators, thiazole vulcanization accelerators, thiuram vulcanization accelerators, thiourea vulcanization accelerators, guanidine vulcanization accelerators, dithiocarbamate vulcanization accelerators, aldehyde amine vulcanization accelerators, aldehyde ammonia vulcanization accelerators, imidazoline vulcanization accelerators, and xanthonic acid vulcanization accelerators. For example, the accelerator may be the accelerator NS (N-tert-butyl-2-benzothiazolesulfenamide). In some embodiments, the raw materials of the rubber composition of the present invention comprise an accelerator, such as the accelerator NS. The raw material of the rubber composition of the present invention may comprise 0 to 1.5 parts by weight, preferably 0.5 to 1.5 parts by weight, more preferably 0.5 to 1.2 parts by weight, such as 0.8± 0.2 parts by weight, 0.8± 0.1 parts by weight of the accelerator, such as the accelerator NS.

In addition, if it is required, the rubber compositions may further comprise plasticizers such as DMP (dimethyl phthalate), DEP (diethyl phthalate), DBP (dibutyl phthalate), DHP (diheptyl phthalate), DOP (dioctyl phthalate), DINP (diisononyl phthalate), DIDP (diisodecyl phthalate), BBP (butyl benzyl phthalate), DWP (dilauryl phthalate), and DCHP (dicyclohexyl phthalate), etc. The amount of the plasticizer may be a routine amount in the art.

The unvulcanized rubber of the present invention may be prepared by a conventional rubber mixing method, for example, it may be prepared by a two-stage mixing method, which comprises mixer mixing in a first stage comprising mixing diene elastomers, reinforcing fillers, additives, and antidegradants to obtain a master batch, and mill mixing in a second stage comprising mixing the master batch obtained in the first stage with a crosslinking agent and an accelerator to obtain an unvulcanized rubber.

The unvulcanized rubber of the present invention may be vulcanized by the conventional vulcanization method to obtain a vulcanized rubber. The vulcanization temperature is usually 130°C to 200°C, such as 140 to 150°C, or 145±2°C. The vulcanization time depends on the vulcanization temperature, vulcanization system, and vulcanization kinetics, and is usually 15 to 60 minutes, such as 20 to 30 minutes, 25±2 minutes. Before vulcanization, conventional tableting can be performed on the kneaded unvulcanized rubber.

The present invention also provides a rubber product, which comprises a rubber composition according to any embodiments of the present invention. Rubber products may be tires, rubber shoes, sealing strips, sound insulation panels, shock absorbing pads, etc. In some embodiments, the rubber product is a tire, such as treads, belt layers, and sidewalls of a tire. The belt layer of the tire, in addition to the rubber composition of the present invention, may also comprise a reinforcing material conventionally used in the art.

The present invention also provides use of the compound of Formula A, the compound of Formula B, the compound of Formula B', the compound of Formula I, the compound of Formula II, or the compound of Formula III in improving the thermal oxidative aging resistance, ozone aging resistance, and/or discoloration resistance of rubber or rubber products. Preferably, the rubber product is a tire. The use comprises adding to rubber or rubber articles the compound of Formula A, the compound of Formula B, the compound of Formula B', the compound of Formula I, the compound of Formula II, or the compound of Formula III according to any embodiments of the present invention as an antidegradant.

The present invention is described in the following specific examples. It should be understood that these examples are merely illustrative and are not intended to limit the scope of the present invention. The methods, reagents, and materials used in the examples, unless otherwise stated, are conventional methods, reagents, and materials in the art. The raw materials used in the examples are commercially available.

### Example 1: Synthesis of Compound I-1 (N-(4-methoxylphenyl)-N'-1,3-dimethylbutyl-1,4-phenylenediamine).

### (1) Synthesis of Compound X-1

200g (1.62mol) p-methoxylaniline, 80ml xylene, and 133.3g (0.37 mol) 25% aqueous solution of tetramethylammonium hydroxide (TMAOH) are added in a 500mL four-mouth flask with stirring and the temperature is raised to 40 to 50°C. TMAOH and p-methoxylaniline react to form salts under distillation and dehydration under reduced pressure. During the process, the color of the reaction liquid gradually changes from yellow to purple-red. The temperature is gradually raised to 70°C. When the amount of fraction is about half of the feeding amount of the 25% tetramethylammonium hydroxide catalyst, at 70°C vacuum (-0.095MPa) distillation and dropwise addition of 41g (0.33mol) nitrobenzene are performed at the same time with the dropwise addition time of about 2hrs. After the dropwise addition, the temperature is kept for 1hr. The reaction is monitored by LC chromatography until nitrobenzene is completely reacted. A condensate liquid is obtained.

The above condensate liquid is transferred to a 500mL stainless steel reactor, to which 30g deionized water and 45g skeleton nickel catalyst are added. The atmosphere is replaced with hydrogen for three times, the temperature is raised to 68°C, and the pressure is raised to 2.0MPa for reaction. The reaction is monitored by LC until nitro-compounds and nitroso-compounds are completely reduced. The reaction liquid is filtered, washed with water, and separated to obtain an organic phase, and the organic phase is distilled under reduced pressure (-0.1MPa, 190°C) to obtain 57g intermediate compound X-1 (yield at about 80%), and its content is >99.4% by GC detection.

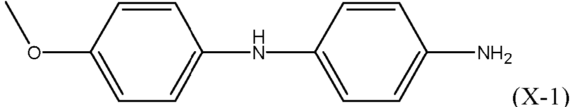

LC-MS(m/z): 214.22(M-H⁺).

### (2) Synthesis of Compound I-1

57g Compound X-1, 150g (1.50mol) 4-methyl-2-pentanone, and 0.6g Pt/C are put into a 500mL high-pressure reactor. The atmosphere is replaced with hydrogen 3 times. The temperature is raised to 75°C, and the pressure is raised to 1.6MPa for reaction, during which hydrogen is replenished in real time. The content of Compound X-1 is <0.1% by GC detection. The temperature is cooled down and the reaction is stopped. The catalyst is removed by filtration, and the light components are removed by distillation under reduced pressure of -0.1MPa and at 170°C to obtain 77.7g of Compound I-1 (yield of about 98%), and its content is >98.5% by GC detection. Characteristic: purple solid.

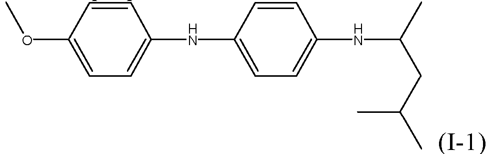

LC-MS(m/z): 298.40 (M-H⁺).

### Example 2: Synthesis of Compound I-2 (N-(4-methoxylphenyl)-N'-cyclohexyl-1,4-phenylenediamine).

### (1) Synthesis of Compound X-1

The synthesis of Compound X-1 is the same as Example 1.

### (2) Synthesis of Compound I-2

40g (0.18mol) Compound X-1, 80g (0.81mol) cyclohexanone, and 0.8g Pt/C are put into a 500mL autoclave reactor. The atmosphere is replaced with hydrogen for three times, the temperature is raised to 100°C, and the pressure is raised to 1.8MPa for reaction, during which hydrogen is replenished in real time. The B1 content is <0.1% by GC detection. The temperature is cooled down and the reaction is stopped. The catalyst is removed by filtration, and the light components are removed by distillation under reduced pressure at -0.1MPa and 200°C to obtain 53.2g Compound I-2 (yield of about 96%), and its content is >97.8%% by GC detection. Characteristic: purple-brown solid.

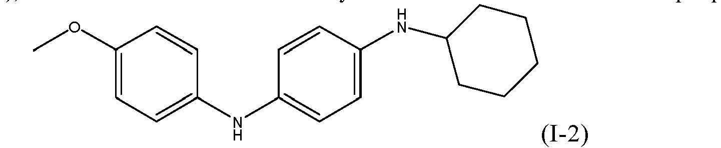

LC-MS(m/z): 296.41 (M-H⁺).

### Example 3: Synthesis of Compound I-3 (N-(2-methoxylphenyl)-N'-1-methylpropyl-1,4-phenylenediamine).

### (1) Synthesis of Compound X-2

400.2g (3.25mol) o-methoxylaniline and 200.2g (0.55mol) 25% aqueous solution of tetramethylammonium hydroxide (TMAOH) are put into a 1000mL four-mouth flask with stirring and the temperature is raised to 40-50°C. TMAOH and o-methoxylaniline are formed into salts by distillation and dehydration under reduced pressure. During the process, the color of the reaction liquid gradually changes from yellow to reddish-brown. The temperature is gradually raised to 75°C. When the amount of fraction is about 100g, 75°C vacuum (-0.097MPa) distillation and dropwise addition of 61.55g (0.50mol) nitrobenzene are performed at the same time with the dropwise addition time of about 3hrs. After the dropwise addition, the temperature is kept for 1hr. The reaction is monitored by LC chromatography until nitrobenzene is completely reacted. A condensate liquid is obtained.

The above condensate liquid is transferred to a 1000mL stainless steel reactor, to which 100g deionized water and 60g skeleton nickel catalyst are added. The atmosphere is replaced with hydrogen for three times, the temperature is raised to 65°C, and the pressure is raised to 1.5MPa for reaction. The reaction is monitored by LC until nitro-compounds and nitroso-compounds are completely reduced. The reaction liquid is filtered, washed with water, and separated. The aqueous phase is concentrated and then reused, and the organic phase is distilled under reduced pressure to obtain 84.6g Intermediate compound X-2 (yield of about 79.5%), and its content is >99.2% by GC detection.

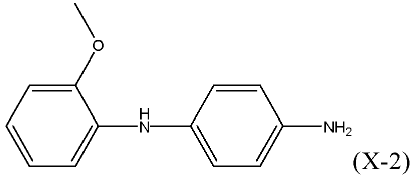

LC-MS(m/z): 214.26(M-H⁺).

### (2) Synthesis of Compound I-3

81.5g (0.38mol) Compound X-2, 117g (1.62mol) 2-butanone, and 1.0g Pt/C are put into a 500mL high-pressure reactor. The temperature is raised to 70°C, the atmosphere is replaced with hydrogen and the pressure is raised to 1.2MPa for reaction. The content of Compound X-2 is <0.1% by GC detection. The temperature is cooled down, and the reaction is stopped. The reaction liquid is filtered, and water and the light components such as 2-butanone are removed by distillation under reduced pressure to obtain 99.5g Compound I-3 (yield at about 97%), and its content is >97.2% by GC detection. Characteristic: purple liquid.

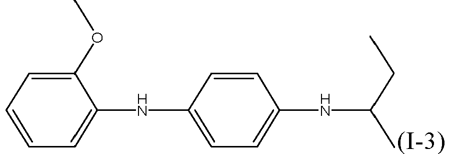

LC-MS(m/z): 270.37 (M-H⁺).

### Example 4: Synthesis of Compound I-4 (N-(2-methoxylphenyl)-N'-1,3-dimethylbutyl-1,4-phenylenediamine).

### (1) Synthesis of Compound X-2

The synthesis method of Compound X-2 is the same as Example 3.

### (2) Synthesis of Compound I-4

50g (0.23mol) Compound X-2, 100g (1.0mol) 4-methyl-2-pentanone, and 1.0g Pt/C are put into a 500mL high-pressure reactor. The temperature is raised to 90°C, the atmosphere is replaced with hydrogen, and the pressure is raised to 1.2MPa for reaction. The content of Compound X-2 is <0.1% by GC detection. The temperature is cooled down and the reaction is stopped. The reaction liquid is filtered, and water and the light components such as 4-methyl-2-pentanone are removed by distillation under reduced pressure to obtain 65.1g of Compound I-4 (yield of about 95%), and its content is >98.2% by GC detection. Characteristic: purple-brown liquid.

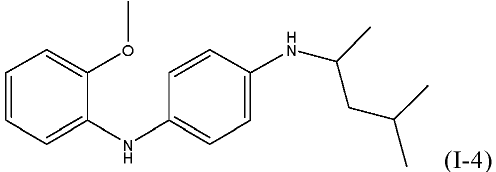

LC-MS(m/z): 298.42(M-H⁺).

### Example 5: Synthesis of Compound I-5 (N-(2-ethoxylphenyl)-N'-1-methylpropyl-1,4-phenylenediamine).

### (1) Synthesis of Compound X-3

220g (1.6mol) o-ethoxylaniline and 80g (0.22mol) 25% aqueous solution of tetramethylammonium hydroxide (TMAOH) are put into a 500mL four-mouth flask with stirring and the temperature is raised to 40 to 50°C. TMAOH and o-ethylethoxylaniline are formed into salts by distillation and dehydration under reduced pressure. During the process, the color of the reaction liquid gradually changes from yellow to reddish-brown. The temperature is gradually raised to 75°C. When the amount of fraction is about 100g, 78°C vacuum (-0.097MPa) distillation and dropwise addition of 24.6g (0.2mol) nitrobenzene are performed at the same time with the dropwise addition time of about 3hrs. After the dropwise addition, the temperature is kept for 1hr. The reaction is monitored by LC chromatography until nitrobenzene is completely reacted. A condensate liquid is obtained.

The above condensate liquid is transferred to a 500mL stainless steel reactor, to which 30g deionized water and 20g skeleton nickel catalyst are added. The atmosphere is replaced with hydrogen for three times, the temperature is raised to 69°C, and the pressure is raised to 1.6MPa for reaction. The reaction is monitored by LC until nitro-compounds and nitroso-compounds are completely reduced. The reaction liquid is filtered, washed with water, and separated. The aqueous phase is concentrated and then reused, and the organic phase is distilled under reduced pressure to obtain 34.1g Intermediate compound X-3 (yield at about 75%), and its content is >98.5% by GC detection.

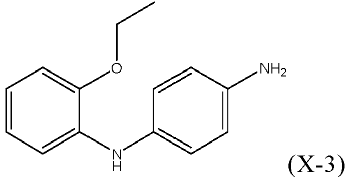

LC-MS(m/z): 228.26(M-H⁺).

### (2) Synthesis of Compound I-5

30g (0.13mol) Compound X-3, 43.2g (0.6mol) 2-butanone, and 0.6g Pt/C are put into a 500mL high-pressure reactor. The temperature is raised to 70°C, the atmosphere is replaced with hydrogen, and the pressure is raised to 1.2MPa for reaction. The content of Compound X-3 is <0.1% by GC detection. The temperature is cooled down, and the reaction is stopped. The reaction liquid is filtered, and water and the light components such as 2-butanone are removed by distillation under reduced pressure to obtain 35.4g Compound I-5 (yield at about 96%), and its content is >97.5% by GC detection. Characteristic: reddish-brown liquid.

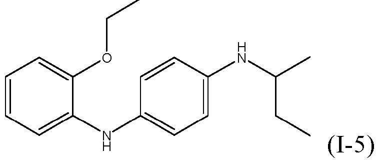

LC-MS(m/z): 284.40 (M-H⁺).

### Example 6: Synthesis of Compound I-6 (N-(2-ethoxylphenyl)-N'-cyclohexyl-1,4-phenylenediamine).

### (1) Synthesis of Compound X-3

The synthesis method of Compound X-3 is the same as Example 5.

### (2) Synthesis of Compound I-6

30g (0.13mol) Compound X-3, 98g (1.0mol) cyclohexanone, and 0.8g Pt/C are put into a 500mL high-pressure reactor. The temperature is raised to 100°C, the atmosphere is replaced with hydrogen, and the pressure is raised to 1.9MPa for reaction. The content of Compound X-3 is <0.1% by GC detection. The temperature is cooled down and the reaction is stopped. The reaction liquid is filtered, and water and the light components such as cyclohexanone are removed by distillation under reduced pressure to obtain 39.5g Compound I-6 (yield t about 98%), and its content is >99.1% by GC detection. Characteristic: dark brown solid.

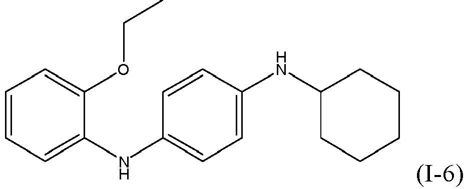

LC-MS(m/z): 310.42(M-H⁺).

### Example 7: Synthesis of Compound I-7 (N-(2-methoxylphenyl)-N'-1-methylpropyl-2-methyl-1,4-phenylenediamine).

### (1) Synthesis of Compound X-4

175.1g (1.42mol) o-methoxylaniline and 87.59g (0.24mol) 25% aqueous solution of tetramethylammonium hydroxide (TMAOH) are put into a 500mL four-mouth flask with stirring and the temperature is raised to 40 to 50°C. TMAOH and o-methoxylaniline are formed into salts by distillation and dehydration under reduced pressure(-0.097MPa). During the process, the color of the reaction liquid gradually changes from yellow to purple red. The temperature is gradually raised to 72°C. When the amount of fraction is about 50% of the feeding amount of 25% tetramethylammonium hydroxide, dropwise addition of 30g (0.22mol) of m-nitrotoluene are performed with the dropwise addition time of about 3hrs. After the dropwise addition, the temperature is kept for 1hr. The reaction is monitored by LC chromatography until m-nitrotoluene is completely reacted. A condensate liquid is obtained.

The above condensate liquid is transferred to a 500mL stainless steel reactor, to which 30g deionized water and 30g skeleton nickel catalyst are added. The atmosphere is replaced with hydrogen for three times, the temperature is raised to 78°C, and the pressure is raised to 2.0MPa for reaction. The reaction is monitored by LC until nitro-compounds and nitroso-compounds are completely reduced. The reaction liquid is filtered, washed with water, and separated. The aqueous phase is concentrated and then reused, and the organic phase is distilled under reduced pressure (-0.1MPa, 190°C) to remove the light components and obtain 40g Intermediate compound X-4 (yield at about 80%), and its content is >99.2% by GC detection.

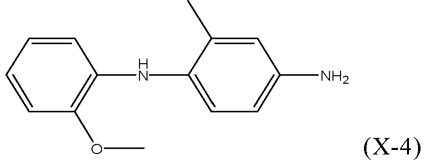

LC-MS(m/z): 228.24(M-H⁺).

### (2) Synthesis of Compound I-7

40g (0.14mol) Compound X-4, 100g (1.38mol) 2-butanone, and 0.5g Pt/C are put into a 500mL reactor. After the atmosphere is replaced with hydrogen for 2 to 3 times, the pressure is raised to 1.2MPa and the temperature is raised to 90°C for reaction. The content of Compound X-4 is <0.1% by GC detection. The temperature is cooled down and the reaction is stopped. The reaction liquid is filtered, and water generated by the reaction and excess of the light components such as 2-butanone are removed by distillation under reduced pressure to obtain 77.5g Compound I-7 (yield at about 97.8%), and its content is >95.9% by GC detection. Characteristic: purple-brown solid.

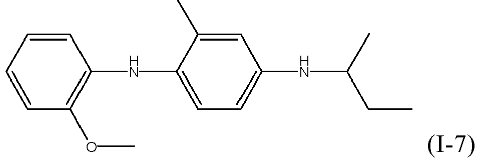

LC-MS(m/z): 284.17 (M-H⁺).

### Example 8: Synthesis of Compound I-8 (N-(2-methoxylylphenyl)-N'-1,3-dimethylbutyl-2-methyl-1,4-phenylenediamine).

### (1) Synthesis of Compound X-4

The synthesis of Compound X-4 is the same as Example 7.

### (2) Synthesis of Compound I-8

30g (0.1mol) Compound X-4, 100g (1mol) 4-methyl-2-pentanone, and 0.6g Pt/C are put into a 500mL reactor. After the atmosphere is replaced with hydrogen for 2 to 3 times, the pressure is raised to 1.5MPa and the temperature is raised to 90°C for reaction. The content of Compound X-4 is <0.1% by GC detection. The temperature is cooled down and the reaction is stopped. The reaction liquid is filtered, and water generated by the reaction and excess of the light components such as 4-methyl-2-pentanone are removed by distillation under reduced pressure to obtain 30.7g Compound I-8 (yield at about 98.5%), and its content is >98.2% by GC detection. Characteristic: deep purple solid.

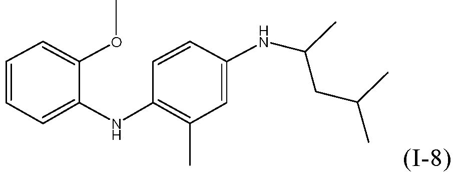

LC-MS(m/z): 312.45 (M-H⁺).

### Example 9: Synthesis of Compound I-9 (N-(2-methoxylylphenyl)-N'-cyclohexyl-2-methyl-1,4-phenylenediamine).

### (1) Synthesis of Compound X-4

The synthesis of Compound X-4 is the same as Example 7.

### (2) Synthesis of Compound I-9

30g (0.1mol) Compound X-4, 60g (0.61mol) cyclohexanone, and 1.0g Pt/C are put into a 500mL reactor. After the atmosphere is replaced with hydrogen for 2 to 3 times, the pressure is raised to 2.0MPa and the temperature is raised to 100°C for reaction. The content of Compound X-4 is <0.1% by GC detection. The temperature is cooled down and the reaction is stopped. The reaction liquid is filtered, and water generated by the reaction and excess of the light components such as cyclohexanone are removed by distillation under reduced pressure (-0.1MPa, 200 °C) to obtain 30.7g Compound I-9 (yield at about 99.1%), and its content is >97.6% by GC detection. Characteristic: dark brown solid.

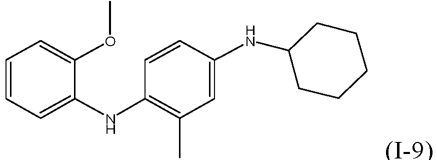

LC-MS(m/z): 310.41 (M-H⁺).

### Example 10: Synthesis of Compound I-10 (N- (4-methoxylphenyl)-N'-cyclohexyl-2-methyl-1,4-phenylenediamine).

### (1) Synthesis of Compound X-5

200g (1.62mol) p-methoxylaniline and 100g (0.27mol) 25% aqueous solution of tetramethylammonium hydroxide (TMAOH) are put into a 500mL four-mouth flask with stirring and the temperature is raised to 40 to 50°C. TMAOH and p-methoxylaniline are formed into salts by distillation and dehydration under reduced pressure (-0.095MPa). During the process, the color of the reaction liquid gradually turns reddish-brown. The temperature is gradually raised to 75°C. When the amount of fraction is about 50% of the feeding amount of 25% tetramethylammonium hydroxide, dropwise addition of 34.2g (0.25mol) m-nitrotoluene are performed with the dropwise addition time of about 3hrs. After the dropwise addition, the temperature is kept for 1hr. The reaction is monitored by LC chromatography until m-nitrotoluene is completely reacted. A condensate liquid is obtained.

The above condensate liquid is transferred to a 500mL stainless steel reactor, to which 20g deionized water and 30g Rainey nickel are added. The atmosphere is replaced with hydrogen for three times, the temperature is raised to 70°C and the pressure is raised to 2.0MPa for reaction, during which hydrogen is continuously replenished. The reaction is monitored by LC until nitro-compounds and nitroso-compounds are completely reduced. The reaction liquid is filtered, washed with water, and separated to obtain an organic phase. The organic phase is distilled under reduced pressure (-0.1MPa, 220 °C) to remove the light components and obtain 45.3 g Intermediate compound X-5 (yield at about 79.5%), and its content is >99.8% by GC detection.

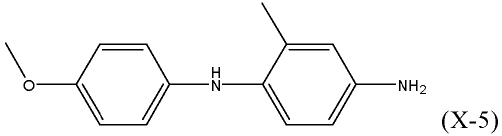

LC-MS(m/z): 228.28(M-H⁺).

### (2) Synthesis of Compound I-10

45.3g (0.15mol) Compound X-5, 98.1g (1.0mol) cyclohexanone, and 1.0g Pt/C are put into a 500mL stainless steel reactor. After the atmosphere is replaced with hydrogen, the pressure is raised to 2.0MPa and the temperature is raised to 75°C for reaction. The content of Compound X-5 is <0.1% by GC detection. The temperature is cooled down and the reaction is stopped. The reaction liquid is filtered, and the light components are removed by distillation under reduced pressure (-0.1MPa, 200 °C) to obtain 59.7g Compound I-10 (yield at about 97%), and its content is >96.8% by GC detection. Characteristic: reddish-brown solid.

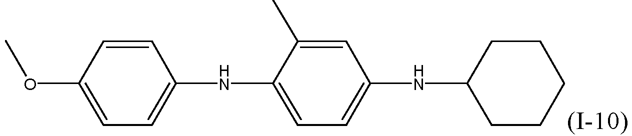

LC-MS(m/z): 310.41 (M-H⁺).

### Example 11: Synthesis of Compound I-11 (N- (4-methoxylphenyl)-N'-phenyl-2-methyl-1,4-phenylenediamine).

### (1) Synthesis of Compound X-5

The synthesis of Compound X-5 is the same as Example 10.

### (2) Synthesis of Compound I-11

30 g (0.1 mol) Compound 10, 9.8 g (0.1 mol) cyclohexanone, 12.3 g (0.11 mol) nitrobenzene, 30 mL toluene, and 1.0 g Pt/C are put into a four-mouth flask equipped with a condenser, water separator, and thermometer, and heated to 110°C for reaction. Dehydration is carried out while the reaction is performed. When the generated water is about to the theoretical amount and the compound X-5 content is <0.1% by GC detection, the reaction is stopped. The light components are removed by filtering and distilling the reaction liquid under reduced pressure (-0.1MPa, 200 °C) to obtain 115.5g Compound I-11 (yield at about 95%). Characteristic: brown solid.

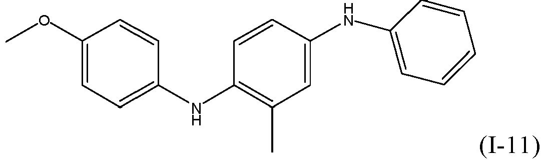

LC-MS(m/z): 304.39 (M-H⁺).

### Example 12: Synthesis of Compound B-1 (N-(3-methylphenyl)-N'-1,3-dimethylbutyl-2-methyl-1,4-phenylenediamine).

### (1) Synthesis of Compound X-6

132.4g (1.23 mol) m-toluidine and 87.6g (0.24 mol) 25% aqueous solution of tetramethylammonium hydroxide (TMAOH) are put into a 500mL four-mouth flask with stirring and the temperature is raised to 40 to 50 °C. TMAOH and m-toluidine are formed into salts by distillation and dehydration under reduced pressure. During the process, the color of the reaction liquid gradually changes from yellow to dark red. The temperature is gradually raised to 72°C. When the amount of fraction is about 50% of the feeding amount of 25% tetramethylammonium hydroxide, 72°C vacuum (-0.098MPa) distillation and dropwise addition of 30g (0.22mol) m-nitrotoluene are performed at the same time with the dropwise addition time of about 3hrs. After the dropwise addition, the temperature is kept for 1hr. The reaction is monitored by LC chromatography until m-nitrotoluene is completely reacted. A condensate liquid is obtained.

The above condensate liquid is transferred to a 500mL stainless steel reactor, to which 50g deionized water and 40g skeleton nickel catalyst are added. The atmosphere is replaced with hydrogen for three times, the temperature is raised to 75°C, and the pressure is raised to 1.5MPa for reaction. The reaction is monitored by LC until nitro-compounds and nitroso-compounds are completely reduced. The reaction liquid is filtered, washed with water, and separated. The organic phase is distilled under reduced pressure (-0.1MPa, 160 °C) to obtain 37.1 g Intermediate compound X-6 (yield at about 80%), and its content is >99.5% by GC detection.

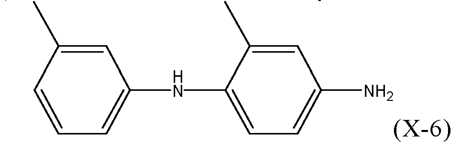

LC-MS(m/z): 212.22(M-H⁺).

¹H NMR (400 MHz, DMSO-*d*₆) δ 6.95 - 6.88 (m, 2H), 6.79 (d, *J =* 8.3 Hz, 1H), 6.48 (d, *J =* 2.6 Hz, 1H), 6.43 - 6.30 (m, 4H), 4.80 (s, 2H), 2.14 (s, 3H), 2.02 (s, 3H).

### (2) Synthesis of Compound B-1

37.1g Compound X-6, 60g (0.60mol) 4-methyl-2-pentanone, and 0.5g Pt/C catalyst are put into the reactor. The atmosphere is replaced with hydrogen for three times, the temperature is raised to 100°C, and the pressure is raised to 1.5MPa for reaction. When the Compound X-6 content is < 0.1% by GC detection, the reaction is stopped. The reaction liquid is cooled down, and the catalyst is removed by filtration. The light components are removed by distillation at -0.1MPa and 180°C to obtain 49.2g Compound B-1 (yield at about 95%), and its content is >98.5% by GC detection. Characteristic: reddish-brown solid.

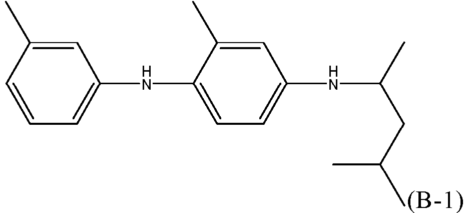

LC-MS(m/z): 296.44(M-H⁺).

### Example 13: Synthesis of Compound B-2 (N-(3-methylphenyl)-N'-1-methylpropyl-2-methyl-1,4-phenylenediamine).

### (1) Synthesis of Compound X-6

The synthesis of Compound X-6 is the same as Example 12.

(2) Synthesis of Compound B-230g (0.14mol) Compound X-6, 100g (1.38mol) 2-butanone, and 0.6g Pt/C catalyst are put into the reactor. The atmosphere is replaced with hydrogen for three times, the temperature is raised to 80°C, and the pressure is raised to 1.5MPa for reaction. When the Compound X-6 content is < 0.1% by GC detection, the reaction is stopped. The reaction liquid is cooled down, and the catalyst is removed by filtration. The light components are removed by distillation at -0.1MPa and 150°C to obtain 36.7g Compound B-2 (yield at about 98%), and its content is >98.5% by GC detection. Characteristic: reddish-brown solid.

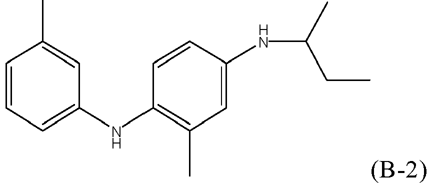

LC-MS(m/z): 268.40 (M-H⁺).

¹H NMR (400 MHz, DMSO-*d*₆) δ 6.96 - 6.87 (m, 2H), 6.83 (d, *J =* 8.4 Hz, 1H), 6.45 (d, *J = 2.6* Hz, 1H), 6.42 - 6.29 (m, 4H), 4.98 (d, *J =* 8.6 Hz, 1H), 2.14 (s, 3H), 2.04 (s, 3H), 1.81 - 1.66 (m, *J* = 6.7 Hz, 1H), 1.45 (dt, *J =* 13.9, 7.1 Hz, 1H), 1.21 (dt, *J* = 13.5, 6.8 Hz, 1H), 0.89 (dd, *J =* 16.2, 6.6 Hz, 6H).

### Example 14: Synthesis of Compound B-3 (N-(2-methylphenyl)-N'-1-methylpropyl-3-methyl-1,4-phenylenediamine).

### (1) Synthesis of Compound X-7

347.9g (3.25mol) o-toluidine and 200g (0.55 mol) 25% aqueous solution of tetramethylammonium hydroxide (TMAOH) are put into a 1000mL four-mouth flask with stirring and the temperature is raised to 60°C. TMAOH and o-toluidine are formed into salts by distillation and dehydration under reduced pressure. During the process, the color of the reaction liquid gradually changes from yellow to reddish-brown. The temperature is gradually raised to 80°C. When the amount of fraction is about 50% of the feeding amount of 25% tetramethylammonium hydroxide, 80°C vacuum (-0.097MPa) distillation and dropwise addition of 68.5g (0.50mol) o-methylnitrobenzene are performed at the same time with the dropwise addition time of about 3hrs. After the dropwise addition, the temperature is kept for 1hr. The reaction is monitored by LC chromatography until o-methylnitrobenzene is completely reacted. A condensate liquid is obtained.

The above condensate liquid is transferred to a 500mL stainless steel reactor, to which 65g deionized water and 38g skeleton nickel catalyst are added. The atmosphere is replaced with hydrogen for three times, the temperature is raised to 78°C, and the pressure is raised to 2.0MPa for reaction. The reaction is monitored by LC until nitro-compounds and nitroso-compounds are completely reduced. The reaction liquid is filtered, washed with water, and separated. The organic phase is distilled under reduced pressure (-0.1MPa, 180 °C) to remove the light components and obtain 31.8 g Intermediate compound X-7 (yield at about 30%), and its content is >92.5% by GC detection.

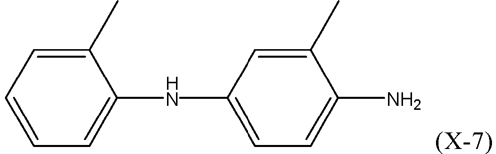

LC-MS(m/z): 212.20(M-H⁺).

(2) Synthesis of Compound B-330g (0.14mol) Compound X-7, 117g (1.62mol) 2-butanone, and 0.8g Pt/C are put into the reactor. The temperature is raised to 80°C, and the pressure is raised to 1.5MPa for reaction after hydrogen replacement. When the content of Compound X-7 is <0.1% by GC detection, it is cooled down and the reaction is stopped. The light components are removed by filtration and vacuum distillation (-0.1MPa, 160 °C) to obtain 36.8g Compound B-3 (yield at about 97%), and its content is >95.5% by GC detection. Characteristic: black solid.

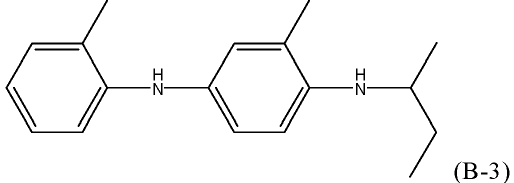

LC-MS(m/z): 268.38 (M-H⁺).

### Example 15: Synthesis of Compound B-4 (N-(2-methylphenyl)-N'-cyclohexyl-3-methyl-1,4-phenylenediamine).

### (1) Synthesis of Compound X-7

The synthesis of Compound X-7 is the same as Example 14.

### (2) Synthesis of Compound B-4

30g (0.14 mol) Compound X-7, 100g (1.02mol) cyclohexanone, and 0.9g Pt/C are put into a 500mL reactor. The temperature is raised to 70°C, and the pressure is raised to 1.8MPa for reaction after hydrogen replacement. When the content of Compound X-7 is <0.1% by GC detection, it is cooled down and the reaction is stopped. The light components are removed by filtration and vacuum distillation (-0.1MPa, 190 °C) to obtain 39.0g Compound B-4 (yield at about 94.8%), and its content is >93.7% by GC detection. Characteristic: black solid.

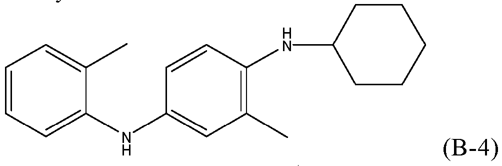

LC-MS(m/z): 294.41 (M-H⁺).

### Example 16: Synthesis of Compound B-5 (N-(2-methylphenyl)-N'-1-methylpropyl-2-methyl-1,4-phenylenediamine).

### (1) Synthesis of Compound X-8

175g (1.6mol) o-toluidine and 100g (0.27 mol) 25% aqueous solution of tetramethylammonium hydroxide (TMAOH) are put into a 1000mL four-mouth flask with stirring and the temperature is raised to 60°C. TMAOH and o-toluidine are formed into salts by distillation and dehydration under reduced pressure. During the process, the color of the reaction liquid gradually changes from yellow to reddish-brown. The temperature is gradually raised to 75°C. When the amount of fraction is about 50% of the feeding amount of 25% tetramethylammonium hydroxide, 80°C vacuum (-0.097MPa) distillation and dropwise addition of 34.2g (0.25mol) of m-methylnitrobenzene are performed at the same time with the dropwise addition time of about 3hrs. After the dropwise addition, the temperature is kept for 1hr. The reaction is monitored by LC chromatography until m-methylnitrobenzene is completely reacted. A condensate liquid is obtained.

The above condensate liquid is transferred to a 500mL stainless steel reactor, to which 35g deionized water and 30g skeleton nickel catalyst are added. The atmosphere is replaced with hydrogen for three times, the temperature is raised to 75°C, and the pressure is raised to 1.5MPa for reaction. The reaction is monitored by LC until nitro-compounds and nitroso-compounds are completely reduced. The reaction liquid is filtered, washed with water, and separated. The organic phase is distilled under reduced pressure (-0.1MPa, 170 °C) to remove the light components and obtain 42.4 g Intermediate compound X-8 (yield at about 80%), and its content is >99.5% by GC detection.

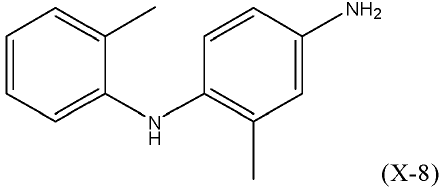

LC-MS(m/z): 212.26(M-H⁺).

### (2) Synthesis of compound B-5

40g (0.18mol) Compound X-8, 100g (1.39mol) 2-butanone, and 0.8g Pt/C are put into a 500mL reactor. The temperature is raised to 78°C, and the pressure is raised to 1.8MPa for reaction after hydrogen replacement. When the content of Compound X-8 is <0.1% by GC detection, it is cooled down and the reaction is stopped. The light components are removed by filtration and vacuum distillation (-0.1MPa, 160 °C) to obtain 47.5g Compound B-5 (yield at about 98%), and its content is >98.5% by GC detection. Characteristic: brown solid.

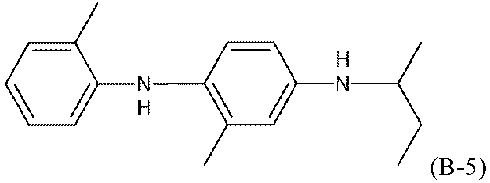

LC-MS(m/z): 268.39 (M-H⁺).

### Example 17: Synthesis of Compound B-6 (N-(2-methylphenyl)-N'-cyclohexyl-2-methyl-1,4-phenylenediamine).

### (1) Synthesis of Compound X-8

The synthesis of Compound X-8 is the same as Example 16.

### (2) Synthesis of Compound B-6

30g (0.14mol) Compound X-8, 100g (1.02mol) cyclohexanone, and 0.9g Pt/C into a 500mL reactor. The temperature is raised to 100°C, and the pressure is raised to 2.0MPa for reaction after hydrogen replacement. When the content of Compound X-8 is <0.1% by GC detection, it is cooled down and the reaction is stopped. The light components are removed by filtration and vacuum distillation (-0.1MPa, 180 °C) to obtain 41.1g Compound B-6 (yield at about 98.8%), and its content is >98.7% by GC detection. Characteristic: dark brown solid.

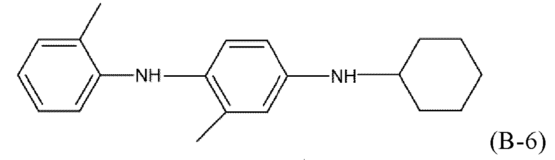

LC-MS(m/z): 294.43 (M-H⁺).

### Example 18: 2-methyl-N-phenyl-1,4-phenylenediamine antidegradant

### (1) Synthesis of Intermediate X-9

176.5 g (1.89 mol) aniline and 116.8 g (0.32 mol) 25% tetramethylammonium hydroxide (TMAOH) are put into a 500 ml four-mouth flask with stirring and the temperature is raised to 40 to 50 °C. TMAOH and aniline are formed into salts by distillation and dehydration under reduced pressure. During the process, the color of the reaction liquid gradually changes from yellow to dark red. The temperature is gradually raised to 70°C. When the amount of fraction is about 50% of the feeding amount of TMAOH, 70 °C vacuum (-0.095MPa) distillation and dropwise addition of 40g (0.29mol) m-nitrotoluene are performed at the same time with the dropwise addition time of about 3hrs. After the dropwise addition, the temperature is kept for 1hr. The reaction is monitored by LC until m-nitrotoluene is completely reacted.

The above condensate liquid is transferred to a 500mL stainless steel reactor, to which 51g deionized water and 30g CAT2 as a catalyst are added. The atmosphere is replaced with hydrogen for three times, the temperature is raised to 75°C, and the pressure is raised to 1.5MPa for reaction. The reaction is monitored by LC until nitro-compounds and nitroso-compounds are completely reduced. The reaction liquid is filtered, washed with water, and separated. The aqueous phase is concentrated and reused. The light components are removed by distilling the organic phase under reduced pressure to obtain the compound 2-methyl-N-phenyl-1,4-phenylenediamine, that is, Intermediate X-9: 45.9 g (single-pass yield at about 79.4%), which is a pink solid with a content of > 99.8% by GC detection.

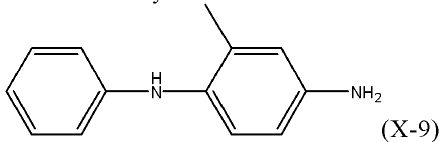

LC-MS(m/z): 198.22(M-H⁺).

¹H NMR (400 MHz, DMSO-*d*₆) δ 7.08 - 6.97 (m, 3H), 6.80 (d, *J =* 8.3 Hz, 1H), 6.57 - 6.46 (m, 4H), 6.40 (dd, *J =* 8.3, 2.7 Hz, 1H), 4.81 (s, 2H), 2.02 (s, 3H).

### (2) Synthesis of Compound B'-1

46 g (0.23 mol) Intermediate X-9, 70 g (0.70 mol) 4-methyl-2-pentanone, 0.8 g Pt/C are put into a 500ml reactor. The atmosphere is replaced with hydrogen for three times, the temperature is raised to 80°C, and the pressure is raised to 1.8MPa for reaction. When the content of Compound X-9 is < 0.1% by GC detection, it is cooled down and the reaction is stopped. The light components are removed by filtration and vacuum distillation (-0.1MPa, 180 °C) to obtain 64.0 g Compound B'-1 (yield at about 98%), and its content is >95.8% by GC detection.

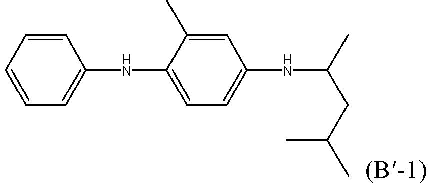

### Characteristic: dark red liquid

LC-MS(m/z):282.40 (M-H⁺).

¹H NMR (400 MHz, DMSO-*d*₆) δ 6.83 (t, *J =* 7.8 Hz, 1H), 6.80 - 6.74 (m, 2H), 6.68 - 6.61 (m, 1H), 6.60 - 6.54 (m, 2H), 6.53 - 6.47 (m, 2H), 4.87 (s, 1H), 3.39 (t, *J =* 6.7 Hz, 2H), 2.55 (s, 1H), 2.21 (s, 3H), 2.07 (s, 3H), 1.80 - 1.58 (m, *J* = 6.8 Hz, 1H), 1.44 (d, *J =* 13.9 Hz, 1H), 1.31 - 1.09 (m, 1H), 1.06 (d, *J=* 6.1 Hz, 3H), 0.89 (dd, *J =* 14.8, 6.6 Hz, 6H).

### (3) Synthesis of Compound B'-2

40g (0.2 mol) Intermediate X-9, 100g (1.39 mol) 2-butanone, 0.5g Pt/C are put into a 500ml reactor. The atmosphere is replaced with hydrogen for three times, the temperature is raised to 80°C, and the pressure is raised to 1.2MPa for reaction. When the content of Compound X-9 is < 0.1% by GC detection, it is cooled down and the reaction is stopped. The light components are removed by filtration and vacuum distillation (-0.1MPa, 190 °C) to obtain 49.4g Compound B'-2 (yield at about 98%), and its content is >97.6% by GC detection.

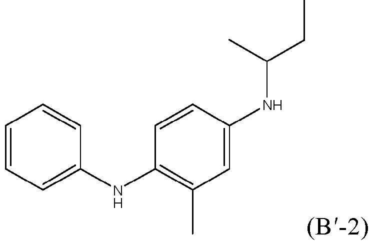

### Characteristic: reddish-brown liquid

LC-MS(m/z): 254.35 (M-H⁺).

### (4) Synthesis of Compound B'-3

40 g (0.2 mol) Intermediate X-9, 100 g (1.39 mol) cyclohexanone, 0.8 g Pt/C are put into a 500ml reactor. The atmosphere is replaced with hydrogen for three times, the temperature is raised to 100°C, and the pressure is raised to 2.0MPa for reaction. When the content of Compound X-9 is < 0.1% by GC detection, it is cooled down and the reaction is stopped. The light components are removed by filtration and vacuum distillation (-0.1MPa, 190 °C) to obtain 54.4g Compound B'-3 (yield at about 97%), and its content is >97.8% by GC detection.

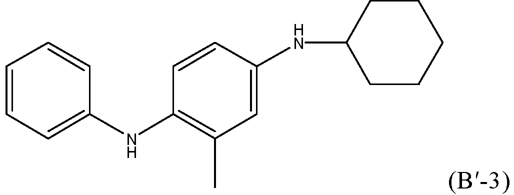

### Characteristic: reddish-brown solid

LC-MS(m/z): 280.41 (M-H⁺).

¹H NMR (400 MHz, DMSO-*d*₆) δ 7.08 - 6.99 (m, 3H), 6.83 (d, *J =* 8.4 Hz, 1H), 6.56 - 6.48 (m, 3H), 6.46 (d, *J =* 2.6 Hz, 1H), 6.39 (dd, *J =* 8.4, 2.7 Hz, 1H), 5.12 (d, *J* = 8.1 Hz, 1H), 3.14 d, *J =* 10.2, 4.5 Hz, 1H), 2.03 (s, 3H), 1.92 (d, *J =* 12.1 Hz, 2H), 1.71 (d, *J =* 11.7 Hz, 3H), 1.64 - 1.50 (m, 1H), 1.40 - 1.01 (m, 6H).

### (3) Synthesis of Compound B'-4

19.8 g (0.1 mol) Intermediate X-9, 9.8 g (0.1 mol) cyclohexanone, 12.3 g (0.11 mol) nitrobenzene, 30 mL toluene, and 1.0 g Pt/C are put into a four-mouth flask equipped with a condenser and a water separator and heated to 110°C for reaction. Dehydration is carried out while the reaction is performed. When the generated water is about to the theoretical amount and the content of Compound X-9 is <0.1% by GC detection, the reaction is stopped. The light components are removed by filtering and distilling the reaction liquid under reduced pressure (-0.1MPa, 180 °C) to obtain 18.2g Compound B'-4 (yield at about 92%).

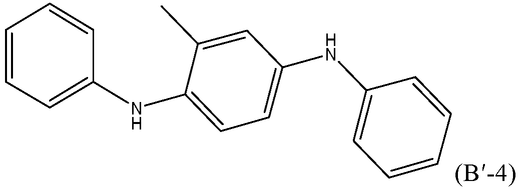

### Characteristic: brown solid

LC-MS(m/z): 274.34 (M-H⁺).

¹H NMR (400 MHz, DMSO-*d*₆) δ 7.96 (s, 1H), 7.26 - 7.11 (m, 4H), 7.11 - 7.05 (m, 2H), 7.05 - 6.98 (m, 3H), 6.97 (d, *J =* 2.6 Hz, 1H), 6.90 (dd, *J =* 8.5, 2.7 Hz, 1H), 6.78 - 6.72 (m, 1H), 6.71 - 6.66 (m, 2H), 6.63 (td, *J =* 7.2, 1.2 Hz, 1H), 2.13 (s, 3H).

### Application Examples

The antidegradant 6PPD and Compound B-1, Compound B-2, Compound I-3, Compound I-4, Compound I-7 and Compound I-8 prepared in the above Examples are used to prepare rubber stocks and the performances of the rubber stocks are tested.

### 1. Raw materials:

Natural rubber (SCR5), Xishuangbanna Sinochem Rubber Co., Ltd.;
Cis-butadiene rubber (BR), Shandong Yuhuang Chemical Co., Ltd.;
Antidegradant 6PPD, Shengao Chemical Technology Co., Ltd.; and
Carbon black N550, aromatic oil, ZnO, stearic acid, sublimation sulfur (S) and accelerator NS are all common raw materials in the rubber industry.

### 2. Equipments and instruments:

FARREL BR1600 Mixer, Farrell Corporation, USA;
X(S)K-160 mill, Shanghai Double Wing Rubber and Plastic Machinery Equipment Co., Ltd.;
63TDF-DSM vulcanizing machine, Huzhou Hongqiao Rubber Machinery Co., Ltd.;
UR2010SD Vulcanizing Instrument, UM2050 Mooney Viscometer, Ucan Technology Co., Ltd.;
Instron 3360 Tensile Machine, Instron Corporation, USA;
CLM-QLH-150 Hot Air Aging Box, Wuxi Colim Environmental Technology Co., Ltd.;
GT-7011-D Flexometer and OZ-0200AC ozone aging tester, Gotech Testing Machines Inc;
and CS-200 colorimeter, Hangzhou Color Spectrum Technology Co., Ltd.

### 3. Rubber stock formulation

**Table 1: Mixing formulation with different antidegradants (unit: parts by mass)**

| Formulation | Rubber Stock 1 | Rubber Stock 2 | Rubber Stock 3 | Rubber Stock 4 | Rubber Stock 5 | Rubber Stock 6 | Rubber Stock 7 |
|---|---|---|---|---|---|---|---|
| SCR5 | 50.0 | 50.0 | 50.0 | 50.0 | 50.0 | 50.0 | 50.0 |
| BR | 50.0 | 50.0 | 50.0 | 50.0 | 50.0 | 50.0 | 50.0 |
| N550 | 50.0 | 50.0 | 50.0 | 50.0 | 50.0 | 50.0 | 50.0 |
| ZnO | 5.0 | 5.0 | 5.0 | 5.0 | 5.0 | 5.0 | 5.0 |
| Stearic acid | 2.0 | 2.0 | 2.0 | 2.0 | 2.0 | 2.0 | 2.0 |
| Aromatic oil | 5.0 | 5.0 | 5.0 | 5.0 | 5.0 | 5.0 | 5.0 |
| 6PPD | 2.0 | | | | | | |
| Compound B-1 | | 2.0 | | | | | |
| Compound B-2 | | | 2.0 | | | | |
| Compound I-3 | | | | 2.0 | | | |
| Compound I-4 | | | | | 2.0 | | |
| Compound I-7 | | | | | | 2.0 | |
| Compound I-5 | | | | | | | 2.0 |
| NS | 0.8 | 0.8 | 0.8 | 0.8 | 0.8 | 0.8 | 0.8 |
| S | 1.5 | 1.5 | 1.5 | 1.5 | 1.5 | 1.5 | 1.5 |
| Total | 166.3 | 166.3 | 166.3 | 166.3 | 166.3 | 166.3 | 166.3 |

### 4. Preparation of rubber stocks

According to the formulation shown in Table 1, SCR5 and BR are first plasticated on a mixer. After they are full mixed, ZnO, stearic acid, antidegradants (Compound B-1, Compound B-2, Antidegradant 6PPD, Compound I-3, Compound I-4, Compound I-7 or Compound I-8), N550 and Aromatic oil are added in sequence and mixed evenly to obtain a master rubber. The master rubber, S and NS are added to a mill. After the rubber stock is mixed evenly, the mill run is performed 5 times, and the roller pitch is adjusted to an appropriate range to obtain an unvulcanized rubber stock.

Standing for about 15 hours, the unvulcanized rubber stock is tested for its vulcanization characteristics, Mooney viscosity and scorching performance.

The unvulcanized rubber stock is vulcanized on the plate vulcanizing machine (145 °C, the vulcanization time is determined according to the vulcanization curve of each antidegradant and between 15~30 min) to obtain a vulcanized rubber stock.

### 5. Performance test

Material inspection and rubber stock performance testing are carried out according to the following standards.

The Mooney viscosity of unvulcanized rubber stock is tested according to GB/T1232.1-2016, and the results are shown in Table 2.

The initial vulcanization characteristics of unvulcanized rubber stock are tested according to GB/T 1233-2008, the scorching time of unvulcanized rubber stock is tested by Mooney instrument (120 °C), and the results are shown in Table 2.

The vulcanization characteristics are tested with rotorless vulcanization instrument for rubber according to GB/T 9869-2014, the vulcanization rate and vulcanization degree of rubber stock are measured by vulcanization meter (145 °C), and the results are shown in Table 2;

The original physical properties (tensile strength, elongation at break) of vulcanized rubber stock are determined according to GB/T 528-2009 rubber, vulcanized or thermoplastic-determination of tensile stress-strain properties, and the results are shown in Table 3.

The thermal oxidative aging resistance of vulcanized rubber stock is determined according to GB/T 13939-2014 rubber, vulcanized or thermoplastic -hot air accelerated aging and heat resistance tests, and the results are shown in Table 3.

According to GB/T 11206-2019 test for rubber deterioration - Surface cracking, the vulcanized rubber stock is tested for static ozone aging performance in the ozone aging test chamber, and the experimental conditions are as follows: a concentration of static ozone of 50pphm, a temperature of 40 °C, a tensile of 20%. Two sets of experiments are carried out for each rubber stock, and the results are shown in Table 4. The meanings represented by 1c, 2c, 3c, and 4c in Table 4 are referred to Standard GB/T 11206-2019.

According to GB/T 13642-2015 rubber, vulcanized or thermoplastic- resistance to ozone cracking- dynamic stain testing, the vulcanized rubber stock is tested for dynamic ozone resistance in the ozone aging test chamber, and the experimental conditions are as follows: a concentration of dynamic ozone of 50pphm, a temperature of 40°C, a dynamic stain of 20%, a frequency of 0.5Hz. Two sets of experiments are carried out for each rubber stock, and the results are shown in Table 5. The meanings represented by 1c, 2c, 3c and 4c in Table 5 are referred to Standard GB/T 11206-2019.

The vulcanized rubber stock is closely fitted with A4 paper, sealed with a transparent sealing bag, placed in the open air for 15 days, and the surface color of A4 paper is determined by a colorimeter. The results are shown in Table 6 and Figure 1.

### 6. Test results

### (1) Processing/vulcanization performance

**Table 2: Processing and vulcanization characteristics of the rubber stocks**

| Item | Antidegradant | | | | | | |
|---|---|---|---|---|---|---|---|
| | Rubber Stock 1 | Rubber Stock 2 | Rubber Stock 3 | Rubber Stock 4 | Rubber Stock 5 | Rubber Stock 6 | Rubber Stock 7 |
| Mooney viscosity | | | | | | | |
| [ML(1+4)100°C] | 50 | 50 | 49 | 50 | 50 | 51 | 50 |

| Scorched data (120°C) | | | | | | | |
|---|---|---|---|---|---|---|---|
| T₅/min | 29.41 | 27.63 | 26.46 | 28.42 | 30.63 | 29.90 | 29.81 |
| T₃₅/min | 31.28 | 30.07 | 28.66 | 30.23 | 32.74 | 32.51 | 32.40 |

| Vulcanization characteristic data (145°C) | | | | | | | |
|---|---|---|---|---|---|---|---|
| (MH-ML)/dNm | 14.1 | 14.2 | 14.1 | 14.7 | 14.7 | 14.3 | 14.3 |
| TC₁₀/min | 9.24 | 8.93 | 8.87 | 9.08 | 9.13 | 9.07 | 9.10 |
| TC₉₀/min | 15.65 | 13.80 | 13.17 | 13.56 | 14.65 | 14.15 | 14.18 |

### (2) Performance before and after thermal oxidative aging

**Table 3: the original physical properties and the thermal oxidative aging resistance of the rubber stock**

| Item | Rubber Stock | | | | | | |
|---|---|---|---|---|---|---|---|
| | Rubber Stock 1 | Rubber Stock 2 | Rubber Stock 3 | Rubber Stock 4 | Rubber Stock 5 | Rubber Stock 6 | Rubber Stock 7 |
| Physical properties before aging | | | | | | | |
| Tensile strength /MPa | 18.9 | 18.6 | 19.0 | 19.1 | 18.4 | 18.5 | 18.7 |
| Elongation at break/% | 534 | 518 | 520 | 505 | 490 | 500 | 503 |

| 100°C, after thermal oxidative aging for 48 h | | | | | | | |
|---|---|---|---|---|---|---|---|
| Tensile strength reduction rate /% | 31.1 | 34.1 | 31.0 | 23.7 | 24.6 | 31.0 | 31.2 |
| Elongation at break reduction rate/% | 37.6 | 33.5 | 39.8 | 31.9 | 32.2 | 39.4 | 39.1 |

### (3) Ozone aging performance

**Table 4: Static ozone aging resistance of the rubber stock**

| Aging time/h | Rubber Stock 1 | | Rubber Stock 2 | | Rubber Stock 3 | | Rubber Stock 4 | | Rubber Stock 5 | | Rubber Stock 6 | | Rubber Stock 7 | |
|---|---|---|---|---|---|---|---|---|---|---|---|---|---|---|
| 24 | 1c | 1c | 1c | 1c | 2c | 1c | 1c | 1c | 1c | 1c | 2c | 2c | 2c | 2c |
| 48 | 2c | 2c | 2c | 2c | 2c | 2c | 2c | 2c | 2c | 2c | 2c | 2c | 2c | 2c |
| 72 | 2c | 2c | 2c | 2c | 2c | 2c | 2c | 2c | 3c | 2c | 2c | 2c | 3c | 3c |
| 96 | 2c | 3c | 3c | 3c | 3c | 3c | 2c | 2c | 3c | 3c | 3c | 3c | 3c | 3c |
| 120 | 3c | 3c | 3c | 3c | 4c | 3c | 3c | 3c | 3c | 3c | 3c | 3c | 4c | 3c |

**Table 5: Dynamic ozone aging resistance of the rubber stock**

| Aging time/h | Rubber Stock 1 | | Rubber Stock 2 | | Rubber Stock 3 | | Rubber Stock 4 | | Rubber Stock 5 | | Rubber Stock 6 | | Rubber Stock 7 | |
|---|---|---|---|---|---|---|---|---|---|---|---|---|---|---|
| 24 | 1c | 1c | 1c | 1c | 1c | 1c | 1c | 1c | 1c | 1c | 1c | 1c | 1c | 1c |
| 48 | 2c | 1c | 2c | 2c | 2c | 2c | 2c | 2c | 2c | 2c | 2c | 2c | 2c | 2c |
| 72 | 2c | 2c | 2c | 3c | 2c | 3c | 2c | 2c | 2c | 2c | 2c | 3c | 2c | 3c |
| 96 | 3c | 3c | 3c | 3c | 3c | 3c | 3c | 3c | 3c | 3c | 2c | 3c | 3c | 3c |
| 120 | 3c | 3c | 3c | 3c | 3c | 4c | 3c | 3c | 3c | 3c | 3c | 4c | 3c | 3c |

### (4) 15-day weathering and discoloration performance

**Table 6: The weathering-resistance and discoloration performance of the rubber stock (aging for 15 days)**

| Sample | Rubber stock 1 | Rubber stock 2 | Rubber stock 3 | Rubber stock 4 | Rubber stock 5 | Rubber stock 6 | Rubber stock 7 |
|---|---|---|---|---|---|---|---|
| ΔL (compared to standard white) | -31.04 | -28.45 | -28.58 | -28.45 | -34.75 | -29.29 | -31.58 |
| Δa (compared to standard white) | 4.40 | 4.30 | 4.34 | 5.70 | 6.01 | 5.10 | 6.44 |
| Δb (compared to standard white)⁾ | 20.35 | 19.63 | 19.38 | 25.63 | 19.57 | 19.76 | 24.38 |
| ΔE (compared to standard white) | 37.37 | 34.83 | 34.80 | 38.71 | 40.34 | 35.70 | 40.41 |
| simulated color (RGB) | R: 187 | **R:** 195 | R: 195 | R: 196 | R: 190 | R: 196 | R: 189 |
| | G: 168 | G: 171 | G: 169 | G: 169 | G: 168 | G: 174 | G: 162 |
| | B: 136 | B: 130 | B: 126 | B: 126 | B: 137 | B: 131 | B: 123 |

As shown in Table 2, the Mooney viscosity, scorching performance, and vulcanization characteristics of the rubber stocks 2-7 containing the compound of Formula A of the present invention are not significantly different from the rubber stock 1 containing 6PPD, indicating that the compound of Formula A of the present invention have little effect on the processing properties and vulcanization characteristics of the rubber stocks.

Table 3 shows that the physical properties before aging of the rubber stocks 2-7 containing the compound of Formula A of the present invention are close to the rubber stock 1 containing 6PPD. After thermal oxidative aged at 100°C for 48hrs, the rubber stock 4 containing Compound I-3 and the rubber stock 5 containing Compound I-4 have significantly lower tensile strength reduction rate and elongation at break reduction rate than the rubber stock 1 containing 6PPD, indicating that the compound of Formula II of the present invention, which is represented by Compound I-3 and Compound I-4, can give rubber more excellent thermal oxidative aging resistance than 6PPD. After thermal oxidative aged at 100°C for 48hrs, the rubber stock 2 containing Compound B-1 has significantly lower elongation at break reduction rate than the rubber stock 1 containing 6PPD, indicating that Compound B-1 can give rubber better thermal oxidative aging resistance. After thermal oxidative aged at 100°C for 48hrs, the rubber stock 3 containing Compound B-2, the rubber stock 6 containing Compound I-7, and the rubber stock 7 containing Compound I-8 have the tensile strength reduction rate and elongation at break reduction rate close to that of the rubber stock 1.

The static and dynamic ozone aging results in Tables 4 and 5 show that the ozone aging resistance of the rubber stocks 2-7 containing the compound of Formula A according to the present invention is comparable to that of the rubber stock 1 containing 6PPD.

The results of weathering and discoloration in Table 6 show that the discoloration resistance of the rubber stocks 4 and 7 is comparable to that of the rubber stock 1, and the discoloration resistance of the rubber stocks 2, 3 and 6 is significantly improved compared with the rubber stock 1, that is, the discoloration performance of Compounds I-3 and I-5 is similar to that of 6PPD, and the discoloration resistance of Compounds B-1, B-2 and I-7 is better than that of 6PPD.

## Claims

1. A compound of Formula A: 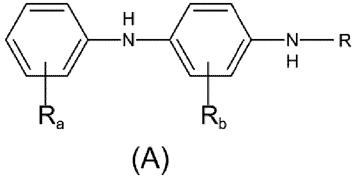
wherein R is selected from a C1-C20 chain hydrocarbon group, a C3-C20 alicyclic hydrocarbon group, a C6-C20 aryl group, and a C1-C20 alkoxy group;
Rₐ is selected from H, a C1-C20 alkyl, a C3-C20 cycloalkyl, phenyl, a C7-C20 alkyl phenyl, a C1-C20 alkyloxy, a C3-C20 cycloalkyloxy, and a C7-C20 alkylphenyloxy;
R_{b} is selected from H, a C1-C20 alkyl, a C3-C20 cycloalkyl, phenyl, and a C7-C20 alkyl phenyl;
and the compound of Formula A does not include compounds where Rₐ is selected from H, a C1-C20 alkyl, a C3-C20 cycloalkyl, phenyl, and a C7-C20 alkyl phenyl, and R_{b} is H.

2. The compound of claim **1,** wherein the compound has a structure represented by Formula B: 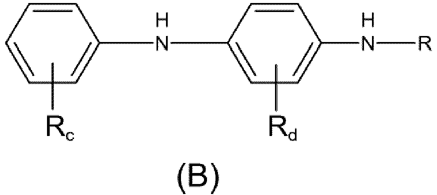
wherein R is selected from a C1-C20 chain hydrocarbon group, a C3-C20 alicyclic hydrocarbon group, and a C6-C20 aryl group; and
R_{c} and R_{d} are independently selected from a C1-C10 alkyl, a C3-C10 cycloalkyl, phenyl, and a C7-C10 alkyl phenyl;
preferably, R is selected from a C3-C10 branched-chain hydrocarbon group, a C3-C10 cycloalkyl group, and a C6-C10 aryl group; more preferably, R is selected from a C4-C6 branched-chain alkyl and a C4-C6 cycloalkyl; and most preferably, R is 1-methylpropyl, 1,3-dimethylbutyl, or cyclohexyl;
preferably, R_{c} and R_{d} are independently selected from a C1-C6 alkyl and a C4-C6 cycloalkyl; and more preferably, R_{c} and R_{d} are independently methyl or ethyl.

3. The compound of claim 1, wherein the compound has a structure represented by Formula I: 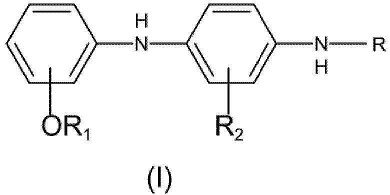
wherein R is selected from a C1-C20 chain hydrocarbon group, a C3-C20 alicyclic hydrocarbon group, a C6-C20 aryl group, and a C1-C20 alkoxy group;
R₁ is selected from a C1-C20 alkyl, a C3-C20 cycloalkyl, and a C7-C20 alkyl phenyl; and
R₂ is selected from H, a C1-C20 alkyl, a C3-C20 cycloalkyl, and a C7-C20 alkyl phenyl;
preferably, R is selected from a C3-C10 branched-chain hydrocarbon group, a C3-C10 cycloalkyl group, and a C6-C10 aryl group; more preferably, R is selected from a C4-C6 branched-chain alkyl, a C4-C6 cycloalkyl and phenyl; and most preferably, R is 1-methylpropyl, 1,3-dimethylbutyl, cyclohexyl, or phenyl;
preferably, R₁ is selected from a C1-C10 alkyl, a C3-C10 cycloalkyl, and a C7-C10 alkyl phenyl; more preferably, R₁ is selected from a C1-C6 alkyl and a C4-C6 cycloalkyl; and most preferably, R₁ is methyl or ethyl;
preferably, R₂ is selected from H, a C1-C10 alkyl, a C3-C10 cycloalkyl, and a C7-C10 alkyl phenyl; more preferably, R₂ is selected from H, a C1-C6 alkyl and a C4-C6 cycloalkyl; and most preferably, R₂ is H, methyl or ethyl.

4. The compound according to claim 3, wherein the compound has a structure represented by Formula II or Formula III, 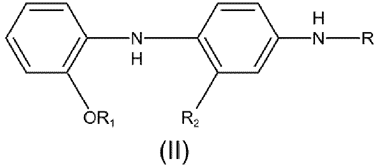 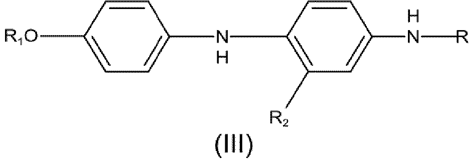 wherein in Formula II and Formula III, R, R₁ and R₂ are as described in claim 3.

5. The compound according to claim 1, wherein the compound is selected from: 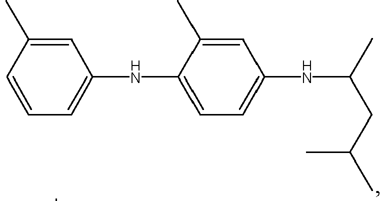 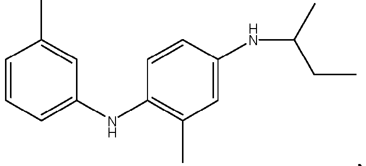 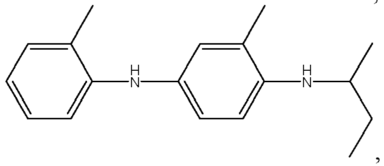 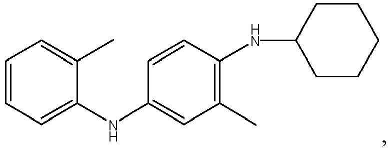 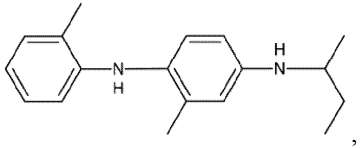 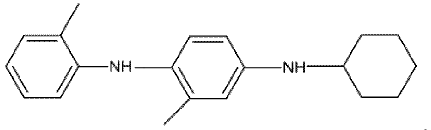 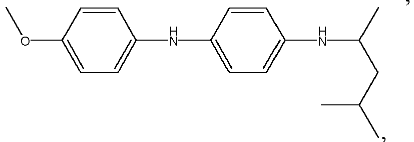 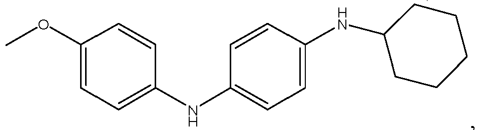 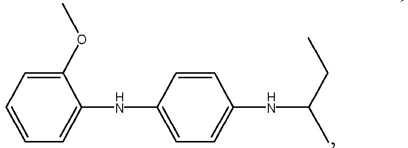 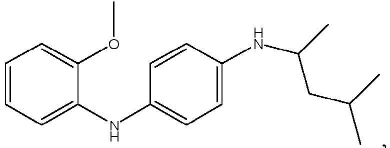 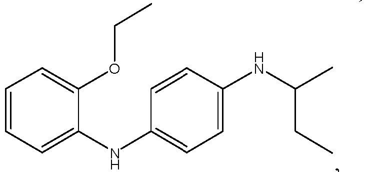 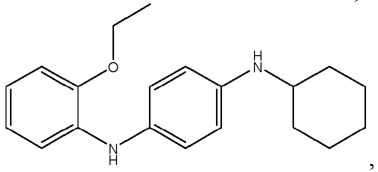 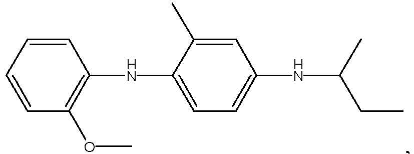 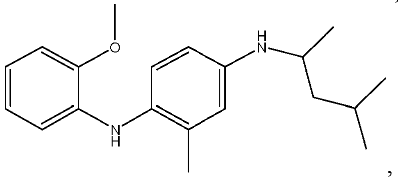 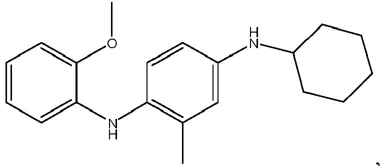 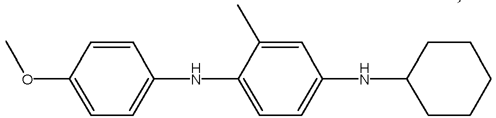 and

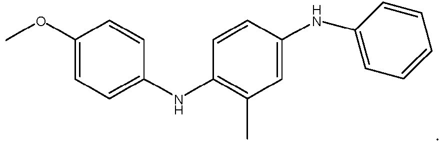

6. A method for preparing the compound according to any one of claims 1 to 5, comprising:
(1) reacting a compound of Formula C and a compound of Formula D in a condensation reaction in presence of a first catalyst to obtain a condensate comprising a compound of Formula E, a compound of Formula F, or both, and subjecting the condensate to a reduction reaction in presence of H₂ and a second catalyst to obtain a compound of Formula X; 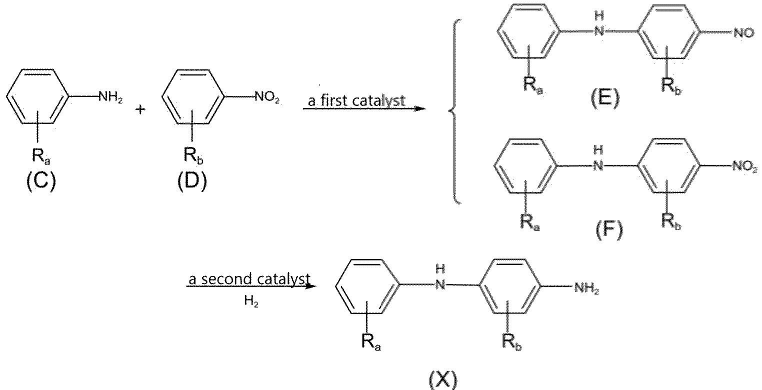
(2) reacting the compound of Formula X with an aldehyde or a ketone in a reductive alkylation reaction in presence of H₂ and a third catalyst to obtain the compound of Formula A; 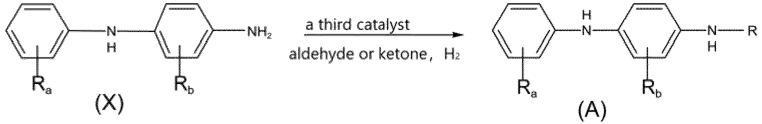 wherein R, Rₐ and R_{b} in Formula C, Formula D, Formula E, Formula F, Formula X and Formula A are as defined in any one of Claims 1 to 5.

7. The method of claim 6, wherein the method has one or more of the following features:
in step (1), the first catalyst is one or more selected from an alkali metal hydroxide, an alkali metal alkoxide, a quaternary ammonium base, and a combination of an alkali metal hydroxide and a halide of tetraalkyl ammonium;
in step (1), a molar ratio of the first catalyst to the compound of Formula C is 0.1: 1 to 2: 1, and preferably 0.9: 1 to 1.1: 1;
in step (1), a molar ratio of the compound of Formula C to the compound of Formula D is 2:1 to 15:1, preferably 4:1 to 10:1, and more preferably 5:1 to 8:1;
a temperature of the condensation reaction of step (1) is 40 to 90°C, and preferably 65 to 85°C;
the condensation reaction of step (1) is carried out under vacuum with a pressure in a range of -0.09 to -0.1MPa;
in step (1), the second catalyst is a porous metal catalyst or a supported metal catalyst; preferably, the porous metal catalyst is one or more selected from Raney nickel, Raney cobalt, and Raney copper; preferably, a metal in the supported metal catalyst is one or more selected from nickel, cobalt, copper, platinum, palladium, ruthenium, and rhodium; and preferably, a support in the supported metal catalyst is one or more selected from carbon, alumina, silica gel, and molecular sieve;
a temperature of the reduction reaction in step (1) is in a range of 40 to 120°C, preferably 60 to 90°C, and a hydrogen pressure is in a range of 0.5 to 5MPa, and preferably 1 to 2MPa;
in step (2), the third catalyst is a supported metal catalyst; preferably, a metal in the supported metal catalyst is one or more selected from nickel, cobalt, copper, platinum, palladium, ruthenium, and rhodium; and a support in the supported metal catalyst is one or more selected from carbon, alumina, silica gel, and molecular sieve;
a molar ratio of the aldehyde or ketone to the compound of Formula X in step (2) is 1:1 to 15:1;
a temperature of the reductive alkylation reaction in step (2) is in a range of 40 to 150°C, and a hydrogen pressure is in a range of 0.5 to 5MPa.

8. A compound of Formula X: 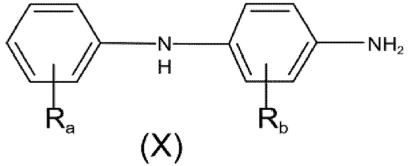 wherein in Formula X, Rₐ and R_{b} are as defined in any one of claims 1 to 5.

9. A method for preparing the compound of Formula X according to claim 8, comprising: step (1) in the method according to claim 6 or claim 7.

10. A rubber composition, comprising the compound according to any one of claims 1 to 5.

11. A rubber product, comprising the rubber composition of claim 10; preferably, the rubber product is a tire.

12. A method for improving thermal oxidative aging resistance, ozone aging resistance, and/or discoloration resistance of rubber or a rubber product, comprising adding the compound according to any one of claims 1 to 5 to the rubber or rubber product.
